# EUROPÄISCHE PATENTSCHRIFT

(11) **EP 2 874 675 B1**
(45) Veröffentlichungstag und Bekanntmachung des Hinweises auf die Patenterteilung: **30.11.2016**
(21) Anmeldenummer: 14716575.7
(22) Anmeldetag: 10.04.2014
(51) Int. Cl.: A61L 29/14, A61L 29/16

(54) **MIT MIKROBOHRUNGEN UND EINEM METALLNETZ VERSEHENER KATHETERBALLON**
CATHETER BALLOON WITH MICRO BOREHOLES AND A METAL MESH
BALLONNET DE CATHÉTER POURVU DE MICROPERÇAGES ET D'UN RÉSEAU MÉTALLIQUE

(30) Priorität: 13.04.2013 EP 13163646
(43) Veröffentlichungstag der Anmeldung: 27.05.2015
(73) Patentinhaber: IPPyramids GmbH, 6370 Stans (CH)
(72) Erfinder: ISSENDORFF, Eberhard, 30880 Rethen (DE)
(74) Vertreter: Arth, Hans-Lothar
(86) Internationale Anmeldenummer: PCT/EP2014/057255
(87) Internationale Veröffentlichungsnummer: WO 2014/167050

(56) Entgegenhaltungen:
- WO-A2-99/55285
- WO-A2-2008/139473

## Beschreibung

Die vorliegende Erfindung betrifft Ballonkatheter mit einem mit Mikrobohrungen und einem Metallnetz versehenen Katheterballon und insbesondere Ballonkatheter für den kardiologischen Bereich zur Verminderung oder Verhinderung von Restenosen. Durch die Mikrobohrungen können Wirkstofflösungen eines Antistenose-Mittels oder eines antiproliferativen, antiangiogenen oder antirestenotischen Wirkstoffs wie bei einem Injektionskatheter appliziert werden, welche vorzugsweise auch zeitgleich für die Dilatation des Katheterballons eingesetzt werden.

Zur Verminderung oder Verhinderung von Restenosen bei einem dilatierten Gefäß, insbesondere einem Blutgefäß, werden im Stand der Technik vorrangig Gefäßstützen, d.h. sogenannte Stents eingesetzt.

Bis heute ist nicht bekannt, wie hoch die Radialfestigkeit von Stents sein muss, um ein Gefäß mit dilatierter Stenose offen zu halten.

Vermutlich sind hierzu nur sehr geringe Kräfte erforderlich, da keine (großen) Kräfte von außen auf die Koronargefäße wirken und ohnehin innen ein Überdruck (Blutdruck) besteht. Vielfach führt allein der (erhöhte) Blutdruck zu Aneurysmen bzw. zu Dissektionen der Gefäßwandung.

Wenn wie häufig üblich einseitige Stenosen mit harten Kalzifizierungen (keine weichen Ablagerungen) vorliegen, wird bei der reinen Ballondilatation das Gefäß nicht gleichmäßig radial geöffnet, sondern asymmetrisch gedehnt als auch häufig überdehnt. Schlimmstenfalls wird die kalzifizierte verkalkte Gefäßseite gar nicht gedehnt und die gegenüberliegende Seite des Gefäßes wird überdehnt oder ruptiert sogar, d.h. Einreißen der Gefäßwandung oder Dissektion.

Die überdehnte (angerissene) bzw. geschädigte Gefäßwand neigt zu einer verstärkten Proliferation und damit zum häufigen Wiederverschluss, was als Restenose bezeichnet wird. Die reine Ballondilatation (ohne Stent) von Koronargefäßen führt deshalb zu einer erhöhten Restenoserate von ca. 30%.

Aus diesem Grund wird eine Ballondilatation vorzugsweise mit Stent durchgeführt, d.h. auf dem Katheterballon ist ein Stent montiert, d.h. gekrimpt, der bei der Ballondilatation mit aufgeweitet wird und bei Deflation des Katheterballons im Gefäß verbleibt.

Zur weiteren Reduzierung der Restenoserate werden oftmals antiinflammatorische, cytostatische, cytotoxische, antiproliferative, antimikrotubuli, antiangiogene, antineoplastische, antimigrative, athrombogene oder antithrombogene Wirkstoffe als anti-Restenosemittel über den Katheterballon verabreicht. Dazu werden die Katheterballons entweder mit dem Wirkstoff oder einer wirkstoffhaltigen Beschichtung beschichtet oder eine Wirkstofflösung wird durch den Katheterballon oder aus Kavitäten im Katheterballon freigesetzt.

Beschichtete Ballonkatheter mit beschichteten Katheterballons als auch Injektionskatheter sind im Stand der Technik bekannt. WO 2010024871 A1 offenbart beispielsweise einen Nadelinjektionskatheter für die Applikation von therapeutischen Wirkstoffen aus Depots, welche in der Ballonhülle ausgebildet sind. Das europäische Patent EP 2269664 B1 schützt Katheterballons, die mit einem Citratester und Paclitaxel beschichtet sind.

Gemäß einiger klinischer Studien kann die reine Ballondilatation entscheidend verbessert werden, wenn gleichzeitig ein Medikament wie z.B. Paclitaxel mit abgegeben wird, welches eine Proliferation des Gefäßes verhindert bzw. den Heilungsprozess begünstigt.

Aktuell gibt es eine Reihe von zugelassenen Ballonkathetern, die außen mit einem Medikament beschichtet sind (beschichtete Ballonkatheter). Für die kurze Zeit, während der Ballon vollständig geöffnet ist (30-120 Sekunden bzw. kürzer und zweimal Öffnen), soll eine gewisse Dosis eines Medikaments in die Gefäßwandung diffundieren und dort für mehrere Tage/Wochen wirken.

Problematisch ist die exakte Bestimmung und Kontrolle der Medikamentenabgabe (Dosierung). Bereits beim Vorschieben des Ballonkatheters durch die Gefäße zur Stenose gehen Teile der Beschichtung durch Diffusion oder gar Delamination verloren. Verlässlich werden nur die Medikamente, die sich in den Ballonfaltungen befinden, bis zur Stenose transportiert. Wie viel Medikament schließlich tatsächlich in die Gefäßwandung diffundiert, hängt zudem von der Dauer der Dilatation und der Ausprägung / Aufnahmefähigkeit des stenosierten Gefäßabschnittes (Kalzifizierung etc.) ab.

Eine Variante von Ballonkathetern für eine besser dosierbare Medikamentenabgabe sind Ballonkatheter, bei denen ein innerer Ballon von einem äußeren umgeben wird. In dem Zwischenraum befindet sich eine Lösung eines Medikaments, welche beim Dilatieren des inneren Ballons aus dem äußeren Ballon, der relativ große Bohrungen enthält, herausgepresst wird.

Zudem sind auch Katheterballons bekannt, welche mit einem Metallnetz überzogen sind. Die Firma TriReme Medical Inc. vertreibt unter der Marke Chocolate^{®} einen Ballonkatheter mit einem Katheterballon, der mit einem Metallgitter überzogen ist.

Ferner offenbart WO 9955285 A2 unter anderem einen sogenannten Scoring-Ballon mit einem Netz als Überzug über den Katheterballon. Das netzartige Gitter kann zum einen auch dazu dienen, einen Wirkstoff freizusetzen und soll zum anderen dazu geeignet sein, Mikrorisse in kalzifizierten Stenosen zu erzeugen, wodurch Gefäßverletzungen und auch Recoil des Gefäßes reduziert werden sollen.

WO 2008/139473 A offenbart eine Vorrichtung mit einem inflatierbaren Ballon zur Abgabe eines bioaktiven Wirkstoffs an Gewebe innerhalb des Körpers, wobei der Wirkstoff beispielsweise durch Mikroporen in dem Katheterballon durch diesen an das Gewebe abgegeben werden kann.

Der Stand der Technik unterscheidet zudem drei Arten von Dilatationsballons, nämlich die sogenannten "compliant"-Ballons, "semi-compliant"-Ballons und "non-compliant"-Ballons.

Die "non-compliant" Ballons bestehen aus einem festen (starren) Material, das sich auch bei hohem Druck nicht oder nur in sehr geringem Maße weiter dehnt. Sie haben einen vorgegebenen Enddurchmesser und werden bei stark kalzifizierten Gefäßstenosen eingesetzt, die eine hohe Kraft respektive einen hohen Druck zum Aufdehnen benötigen. Bricht die Stenose auf und öffnet sich das Gefäß, so kann eine Überdehnung des Gefäßes vermieden werden.

Die "semi-compliant" Ballons weisen ab einem gewissen Druck (z.B. ab einem Druck innerhalb des Bereichs von 6 MPa bis 10 MPa) eine leichte Dehnbarkeit auf. Der Kardiologe kann damit den Ballon durch die Wahl des Druckes an den Gefäßdurchmesser anpassen. Bei harten Stenosen ist aber nicht sichergestellt, dass sich der Ballon über die Länge des stenosierten Gefäßes gleichmäßig auf den Enddurchmesser aufdehnt.

"Compliant" Ballons sind solche, die sich nahezu beliebig je nach Druck dehnen. Derartige Ballons werden in aller Regel nicht für die Gefäßdilatation eingesetzt, da ihre Aufdehnung über die Länge des Ballons nicht kontrollierbar ist. Gegebenenfalls dehnen sie sich nur vor und hinter einer Stenose und überdehnen zudem dort das Gefäß.

Aufgabe der vorliegenden Erfindung ist es nun, einen Ballonkatheter für die Wirkstoffapplikation bereitzustellen, der insbesondere kalzifizierte Stenosen gleichmäßig öffnet und dabei die Überdehnung des nicht kalzifizierten Teils des Gefäßes vermeidet und zudem in der Lage ist, eine definierte Menge eines Antirestenosewirkstoffs abzugeben.

Diese Aufgabe wird erfindungsgemäß durch die technische Lehre des unabhängigen Anspruchs gelöst. Weitere vorteilhafte Ausgestaltungen der Erfindung ergeben sich aus den abhängigen Ansprüchen, der Beschreibung, den Figuren sowie den Beispielen.

Somit betrifft die vorliegende Erfindung einen Ballonkatheter mit einem Katheterballon, wobei der Katheterballon Mikrobohrungen aufweist und mit einem Metallnetz überzogen ist und der Katheterballon mit einer Wirkstofflösung befüllbar und dilatierbar ist und ein vorbestimmtes Volumen der Wirkstofflösung durch die Mikrobohrungen während der Dilatation freigesetzt wird, wobei es sich bei der Wirkstofflösung um eine Lösung eines Antistenose-Mittels oder eines antiproliferativen, antiangiogenen oder antirestenotischen Wirkstoffs handelt.

Offenbart wird ein Ballonkatheter mit einem Katheterballon, wobei der Katheterballon Mikrobohrungen aufweist und mit einem Metallnetz überzogen ist und der Katheterballon zur Aufnahme einer Wirkstofflösung eines Antistenose-Mittels oder eines antiproliferativen, antiangiogenen oder antirestenotischen Wirkstoffs ausgestaltet ist, welche durch die Mikrobohrungen applizierbar ist.

Erfindungsgemäß ist ein Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und dilatierbar ist und ein vorbestimmtes Volumen der Wirkstofflösung durch die Mikrobohrungen während der Dilatation freisetzbar ist.

Bei dem Ballonkatheter handelt es sich vorzugsweise um einen Dilatationskatheter, einen PTCA-Katheter (Percutaneous Transluminal Coronary Angioplasty Katheter), einen PTA-Katheter, einen Infusionskatheter, einen Injektionskatheter oder einen Angioplastiekatheter insbesondere für Blutgefäße und weiter bevorzugt für koronare Blutgefäße.

Ferner handelt es sich erfindungsgemäß bei dem Katheterballon um einen "semi-compliant" oder "non-compliant" Katheterballon aber auch, entgegen der derzeitigen klinischen Anwendung um einen "compliant" Katheterballon.

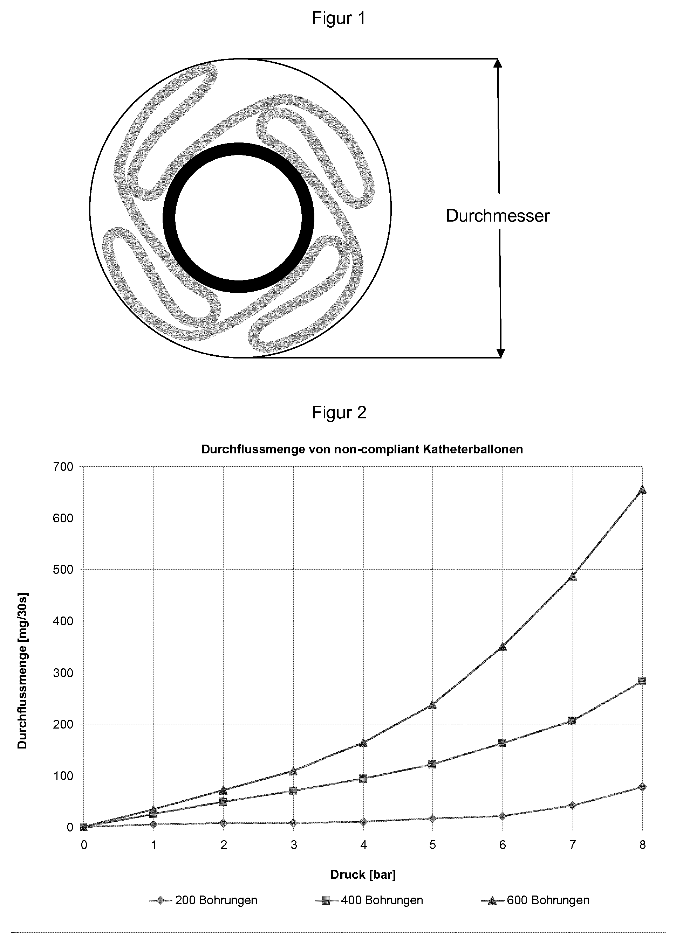

Ein Katheterballon ist dadurch gekennzeichnet, dass der Ballon im komprimierten oder deflatierten Zustand vorzugsweise in Falten liegt und einen anfänglichen möglichst kleinen Durchmesser hat (s. Figur 1). Bei der Dilatation wird im Innern des Katheterballons ein Druck aufgebaut und der Katheterballon vergrößert seinen Durchmesser, indem er sich entfaltet.

Die Ballonhülle weitet sich bei einem **"semi-compliant"** Katheterballon zunächst nicht. Erst nach vollständiger Entfaltung und Erreichung eines Durchmessers, der bei einem Druck (dem sogenannten Nominaldruck) vorliegt, der ausreicht, den Katheterballon vollständig zu entfalten, findet bei weiterer Druckerhöhung eine beschränkte weitere Aufweitung des Katheterballons statt, weil sich die Ballonhülle in einem vorgegebenen Rahmen dehnen kann.

Ein "semi-compliant" PTCA-Katheterballon vergrößert somit seinen gefalteten anfänglichen Durchmesser von vorzugsweise 0,7 - 0,9 mm bei Nominaldruck um das ca. 2,5-fache bis ca. 6,0-fache auf ca. 1,5 bis 5 mm aufgrund der Entfaltung. Nach vollständiger Entfaltung kann eine weitere Vergrößerung des Durchmessers um typischerweise 10% - 20 % (bezogen auf den Durchmesser im vollständig entfalteten Zustand) bei Drücken oberhalb des Nominaldruckes erfolgen aufgrund der Dehnung der Ballonhülle.

Als Durchmesser wird der Außendurchmesser bezeichnet, also von der einen Oberfläche des Katheterballons zur gegenüberliegenden Oberfläche des Katheterballons bei einer idealisierten runden Form so wie in Figur 1 gezeigt.

Somit ist ein bestimmter Druck erforderlich, um den Katheterballon vollständig zu entfalten. Der minimal erforderliche Druck, um eine vollständige Entfaltung zu erreichen, wird auch als Nominaldruck bezeichnet. Eine weitere Vergrößerung des Durchmessers über den bei vollständiger Entfaltung vorliegenden Durchmessers wird durch eine Erhöhung des Drucks über den Nominaldruck erreicht.

In der Regel sind Drücke von 5 MPa - 8 MPa notwendig, um eine vollständige Entfaltung des "semi-compliant" PTCA-Katheterballons zu bewirken. Oberhalb eines Druckes von 5 - 8 MPa beginnt sich die Ballonhülle weiter zu dehnen wie bei einem Luftballon und der Katheterballon vergrößert seinen Durchmesser weiter in einem bestimmten Rahmen, weil es sich um einen "semi-compliant" Katheterballon und nicht um einen "compliant" Katheterballon handelt, der bei weiterer Druckerhöhung seinen Durchmesser weiter vergrößern würde, bis er platzt.

Ein **"compliant"** PTCA-Katheterballon wird im Stand der Technik für die Gefäßdilatation zumeist nicht eingesetzt, da seine Ballonhülle beliebig und unkontrollierbar dehnbar ist, typischerweise bis 100 % aber auch bis zu 500% des Nominaldurchmessers. Er besteht aus einem gummiartigen, stark dehnbaren Material und weist nur einen sehr geringen Nominaldruck bis zu 2 bar (2 MPa) auf.

Ein "compliant" PTCA-Katheterballon vergrößert seinen gefalteten anfänglichen Durchmesser von vorzugsweise 0,7 - 0,9 mm bei Nominaldruck um das ca. 2,5-fache bis ca. 6,0-fache auf ca. 1,5 bis 5 mm aufgrund der Entfaltung. Durch Druckerhöhung kann jedoch eine weitere Vergrößerung des Durchmessers durch Dehnung der Ballonhülle bis zum Berstdruck des Katheterballons erfolgen, wodurch "compliant" PTCA-Katheterballons in der Praxis keine Verwendung gefunden haben. Erfindungsgemäß wird der Einsatz jedoch ermöglicht, da das Metallnetz nur eine begrenzte, durch die Netzstruktur vorgegebene maximale Dehnung zulässt. Das Metallnetz muss dabei so engmaschig sein, dass die Ballonhülle nicht kissenförmig zwischen den Netzstreben herausgedrückt werden kann und vor den Netzstreben liegt. Der Ballondruck kann deutlich bis auf die Werte des "semi-compliant" Ballons erhöht werden. Dabei gilt: je engmaschiger das Metallnetz, desto höher der wählbare Ballondruck.

Beim Nominaldruck ist der "compliant" PTCA-Katheterballon also vollständig entfaltet und das ihn umgebende Metallnetz hat zu einer gleichmäßigen Entfaltung geführt, wodurch auch kalzifizierte Stenosen ohne Überdehnung des nicht kalzifizierten Gefäßabschnittes aufgeweitet werden. Das umgebende Metallnetz ist beim Nominaldruck vollständig oder fast vollständig aufgeweitet und eine weitere Druckerhöhung innerhalb des Katheterballons führt zu einer geringfügigen Dehnung der Ballonhülle, welche aber durch das maximal aufgeweitete Metallnetz eingeschränkt wird, wobei das Metallnetz derart engmaschig ist, dass sich die Ballonhülle nicht durch die Maschen drücken kann. Somit führt eine weitere Druckerhöhung nicht mehr zu einer weiteren, theoretisch möglichen, aber durch das Metallnetz verhinderten Dehnung der Ballonhülle, wobei aber diese weitere Druckerhöhung gezielt für die Applikation einer Wirkstofflösung eines Antistenose-Mittels oder eines antproliferativen, antiangiogenen oder antirestenotischen Wirkstoffs durch die Mikrobohrungen hindurch genutzt werden, wie unten weiter ausgeführt wird.

Sofern erfindungsgemäß ein **"non-compliant"** PTCA-Katheterballon eingesetzt wird, so findet eine Dehnung der Ballonhülle nicht oder nur minimal statt. Der Katheterballon wird unter Druck inflatiert und entfaltet sich bei diesem Vorgang bis er beim Nominaldruck seinen maximalen Durchmesser erreicht. Beim Nominaldruck ist vorzugsweise zudem auch das Metallnetz vollständig aufgeweitet und hat zu einer gleichmäßigen Aufweitung einer kalzifizierten Stenose geführt. Die erfindungsgemäßen Ballonkatheter sind insbesondere zur Dilatation von kalzifizierten und stark kalzifizierten Stenosen geeignet, weil sie ohne Überdehnung des nicht kalzifizierten Gefäßbereiches innerhalb der Stenose zu einer gleichmäßigen Aufweitung des kalzifizierten und stenosierten Gefäßabschnittes führen. Eine weitere Druckerhöhung über den Nominaldruck hinaus führt bei einem "non-compliant" PTCA-Katheterballon nicht zu einer weiteren, wenn auch nur geringen Vergrößerung des Durchmessers, sondern nur zu einer Steigerung des Druckes im Inneren des Katheterballons. Diese weitere Druckerhöhung kann aber gezielt für die Applikation einer Wirkstofflösung eines Antistenose-Mittels oder eines antiproliferativen, antiangiogenen oder antirestenotischen Wirkstoffs durch die Mikrobohrungen hindurch genutzt werden, wie unten weiter ausgeführt wird.

Erfindungsgemäß ist der Katheterballon mit **Mikrobohrungen** versehen, wobei die Mikrobohrungen durchgehende Mikrobohrungen als auch Mikrosacklöcher umfassen. Die durchgehenden Mikrobohrungen sind tatsächliche Mikroöffnungen in der Ballonhülle, wohingegen die Mikrosacklöcher Mikrobohrungen sind, welche die Ballonhülle nicht vollständig durchdringen. Die durchgehenden Mikrobohrungen durchdringen daher die Ballonhülle und ermöglichen den Durchfluß einer Wirkstofflösung bereits bei niedrigeren Drücken, wohingegen die Mikrosacklöcher Öffnungen in der Ballonhülle sind, deren Boden zum Balloninnenlumen hin gerichtet ist und mit einer dünnen Membran verschlossen ist, welche erst ab Erreichung eines gewissen Druckes platzt, wodurch durchgehende Mikrobohrungen erzeugt werden, welche den Austritt von Wirkstofflösung erlauben.

Somit ist gemäß eines Aspekts der vorliegenden Erfindung der Katheterballon mit **durchgehenden Mikrobohrungen** versehen, welche einen Durchmesser haben, dass keine bzw. nur eine geringe Menge Flüssigkeit aus diesen durchgehenden Mikrobohrungen austreten kann, bis nicht ein Minimaldruck erreicht ist, der vorzugsweise dem Nominaldruck entspricht. Dies bedeutet, dass aus dem Inneren des Katheterballons bis zu einem bestimmten Druck keine bzw. keine nennenswerte Menge an Flüssigkeit oder Lösung durch die durchgehenden Mikrobohrungen strömen kann und erst ab einem gewissen Druck die durchgehenden Mikrobohrungen für die Flüssigkeit oder Lösung passierbar werden. Dieser wesentliche Aspekt der Erfindung wird im Folgenden näher beschrieben.

Die Ballonhülle des Katheterballons ist erfindungsgemäß mit Mikrobohrungen versehen, welche vorzugsweise senkrecht zur Längsachse des Katheterballons durch die Ballonhülle verlaufen und ermöglichen, dass eine Flüssigkeit aus dem Inneren des Katheterballons durch diese Mikrobohrungen hindurch nach außen treten kann. Der Durchtritt einer Wirkstofflösung aus dem Balloninneren durch die Mikrobohrungen nach außen kann bei den durchgehenden Mikrobohrungen theoretisch immer erfolgen und bei den Mikrosacklöchern erst nach Bersten der Membran am Boden der Mikrosacklöcher.

Bei den Mikrobohrungen handelt es sich um definierte Bohrungen, welche mittels Laser erzeugt wurden und vorzugsweise eine runde Form besitzen. Es handelt sich bei den Mikrobohrungen jedoch nicht um Kanäle in einem porösen Ballonmaterial und auch nicht um andere nicht geradlinig verlaufende Kanäle in der Ballonhülle. Zudem sind die einzelnen Mikrobohrungen auch nicht miteinander verbunden, so dass ein dreidimensionales Kanalnetzwerk entstünde. Entscheidend ist, dass es sich um definierte und reproduzierbare Formen bei den Mikrobohrungen handelt, welche nachträglich in das Material der Ballonhülle eingebracht wurden und nicht um Strukturen, welche bei der Herstellung der Ballonhülle entstanden sind, wie z.B. poröse Strukturen oder Fehlstellen.

Für die Erzeugung der Mikrobohrungen wird vorzugsweise ein Ultra-Kurz-Pulslaser (UKP-Laser) und alternativ ein Excimer-Laser eingesetzt. Erst mit diesen Lasern neuerer Bauart ist es möglich, derart kleine Bohrungen in die Ballonhülle zu bohren. Diese Laser haben extrem kurze Pulsdauern (Nano-, Piko-, Femtosekunden), sehr hohe Pulsspitzleistungen und eine kurze Wellenlänge der Laserstrahlung typischerweise im UV-Bereich. Erst die Kombination dieser Laserstrahleigenschaften ermöglicht Bohrungen in hauchdünnen Kunststoffen mit Durchmessern wie z.B. den Katheterballonhüllen von wenigen Mikrometern ohne nennenswerte thermische negative Einflüsse der Bohrungsrandzonen oder auf die Ballonhüllen an sich.

Alternativ können die Bohrungen auch durch Ionenstrahlbohren (s. Patente US 6585926 B1 und US 7115299 B2) hergestellt werden. Beim Ionenstrahlbohren ist aber die Positionierung der Mikrobohrungen nur eingeschränkt kontrollierbar.

Es hat sich im Stand der Technik mit Ballonen ohne Metallnetz gezeigt, dass je nach Ballontyp Probleme hinsichtlich des Durchmessers der Mikrobohrungen und der eigentlichen Medikamentenabgabe existieren.

Bei einem non-compliant Ballon ist fast keine Materialdehnung der Ballonhülle möglich. Folglich müssen die Mikrobohrungen so groß sein, dass Flüssigkeit ausströmen kann. Bei fester Bohrungsgröße strömt mehr Flüssigkeit durch die Mikrobohrungen je höher der Druck ist. Hier ist es erforderlich, einen Durchmesser der Mikrobohrungen zu wählen, welcher sicher stellt, dass bei der Dilatation des Katheterballons bis zum Nominaldruck keine oder möglichst wenig Flüssigkeit durch die Mikrobohrungen strömt und erst bei einem Druck gleich oder größer dem Nominaldruck die Mikrobohrungen die Flüssigkeit im Innern des Katheterballons passieren lassen. Die erfindungsgemäßen durchgehenden Mikrobohrungen sind daher so ausgelegt, dass sie bei einem Druck unterhalb des Nominaldruckes keine oder fast keine Flüssigkeit, wie z.B. eine wirkstoffenthaltende Kontrastmittellösung hindurch lassen, d.h. die durchgehenden Mikrobohrungen sind weitgehend verschlossen und werden erst bei einem Druck im Bereich des Nominaldruckes passierbar für eine Flüssigkeit. Die Menge an Wirkstoff bzw. das Volumen an wirkstoffenthaltender Lösung, welche appliziert werden soll, kann durch den Druck im Bereich des Nominaldruckes und die Dauer der Applikation gesteuert werden. Liegt der Nominaldruck beispielsweise bei 10 MPa, so wird bei diesem Druck während der Dilatation innerhalb eines bestimmten Zeitintervalls (z.B. 30 Sekunden) eine bestimmte Menge an Wirkstoff in einem bestimmten Volumen an Kontrastmittellösung appliziert. Soll mehr Wirkstoff appliziert werden, dann kann der Druck über den Nominaldruck z.B. auf 11 MPa erhöht werden, so dass innerhalb desselben Zeitintervalls mehr Kontrastmittellösung durch die Mikrobohrungen hindurch fließt. Bei weiterer Druckerhöhung steigt das Volumen an applizierter wirkstoffenthaltender Kontrastmittellösung weiter, so dass über diese Drucksteuerung in Verbindung mit der Dilatationsdauer und der Konzentration des Wirkstoffs im Kontrastmittel eine genau definierte Wirkstoffmenge appliziert werden kann. Dies ist in dieser Weise mit keinem PTCA-Katheterballon aus dem Stand der Technik möglich.

Bei einem compliant-Ballon (entsprechend dem Stand der Technik ohne Metallnetz) ist es grundsätzlich sehr schwierig eine gleichmäßige Aufweitung des Ballons zu bewerkstelligen, so dass "compliant"-Ballons in der Praxis bisher keine Anwendung für Dilatationskatheter gefunden haben.

Bei dem "semi-compliant" und "compliant" Ballon ist die Wahl der **Bohrungsgröße** wichtig, da sich die Mikrobohrungen unter Druck weiten. Sind die Mikrobohrungen zu klein, muss ein hoher Druck aufgebaut werden (damit Wirkstoff abgegeben wird), womit das Gefäß gegebenenfalls überdehnt wird. Sind die Mikrobohrungen zu groß, wird bei einem benötigten hohen Ballondruck zuviel Wirkstoff abgegeben. Die richtige Größe, d.h. den richtigen Durchmesser für die Mikrobohrungen zu finden, ist daher erfindungswesentlich. Da sich die Bohrungen bei den compliant und semi-compliant Ballonen unter Druck weiten, sind die Bohrungsdurchmesser kleiner zu wählen als bei den non-compliant Ballonen, deren Bohrungen sich nicht unter Druck weiten.

Dieses im Stand der Technik für Injektionsballone bekannte Problem wird durch das erfindungsgemäß vorgesehene Netz verhindert. Das Metallnetz kann bis zu einem bestimmten Außendurchmesser aufgeweitet werden und umhüllt den dehnbaren Katheterballon derart fest, dass eine weitere Druckerhöhung nicht zu einer weiteren Dehnung der Ballonhülle führen kann. Somit kann eine Überdehnung des Gefäßes oder eine ungleichmäßige Dehnung eines kalzifizierten Gefäßabschnitts vermieden werden und eine weitere Drucksteigerung erhöht hingegen das Volumen an durch die Mikrobohrungen hindurch applizierte Wirkstofflösung.

Die einzelnen Mikrobohrungen und vorzugsweise die durchgehenden Mikrobohrungen haben erfindungsgemäß einen Innendurchmesser von 0,5 µm - 5,0 µm, bevorzugt von 0,6 µm - 4,0 µm, weiter bevorzugt von 0,7 µm - 3,6 µm, noch weiter bevorzugt von 0,8 µm - 3,3 µm, noch weiter bevorzugt von 0,9 µm - 3,0 µm, noch weiter bevorzugt von 1,0 µm - 2,5 µm, noch weiter bevorzugt von 1,1 µm - 2,4 µm, noch weiter bevorzugt von 1,2 µm - 2,3 µm, noch weiter bevorzugt von 1,3 µm - 2,2 µm, noch weiter bevorzugt von 1,4 µm - 2,1 µm und am meisten bevorzugt von 1,5 µm - 2,0 µm. Der Innendurchmesser bezieht sich auf eine idealisiert runde Form der Mikrobohrungen. Sofern der Innendurchmesser über die Tiefe der Mikrobohrung nicht konstant sein sollte, wird als Innendurchmesser der kleinste Innendurchmesser bezeichnet.

Erfindungswesentlich sind daher folgende Merkmale für die durchgehenden Mikrobohrungen:
a) Die Mikrobohrungen werden so klein gewählt, dass eine nennenswerte Wirkstoffabgabe erst bei einem hohen Druck erfolgt.
b) Eine Überdehnung des Gefäßes (durch einen zu stark druckgeweiteten Katheterballon) wird durch das Metallnetz, z.B. ein NiTi-Stützgerippe verhindert, welches nur eine maximale vorgegebene Größe, d.h. einen maximalen Durchmesser des dilatierten Katheterballons zulässt.
c) Beim Druckabbau während der Deflation des Katheterballons und insbesondere eines semi-compliant oder compliant Katheterballons ziehen sich die Mikrobohrungen wieder auf den ursprünglichen Durchmesser zusammen und lassen keinen weiteren Austritt einer wirkstoffhaltigen Lösung mehr zu bzw. nur einen sehr geringen.

Die vorliegende Erfindung betrifft somit auch einen Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und/oder dilatierbar ist und die Wirkstofflösung durch die Mikrobohrungen applizierbar ist und die Mikrobohrungen einen Durchmesser von 0,5 µm bis 20 µm aufweisen und der Katheterballon pro mm² 0,2 bis 10 Mikrobohrungen aufweist.

Die Mikrobohrungen befinden sich dabei vorzugsweise nur in dem zylindrischen Teil des Katheterballons und nicht an den beiden Enden. Vorzugsweise sind die Mikrobohrungen in dem mittleren Bereich des Katheterballons weitgehend gleichmäßig verteilt. Der mittlere Bereich ist als 80% der Gesamtlänge des Katheterballons definiert und 10% der Gesamtlänge am Anfang und 10% der Gesamtlänge am Ende des Katheterballons werden als die Randbereiche definiert, welche deutlich weniger (also nur noch 50% bis 1 % der Anzahl an Mikrobohrungen im zylindrischen mittleren Bereich) oder keine Mikrobohrungen mehr aufweisen.

Des weiteren betrifft die vorliegende Erfindung einen Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und/oder dilatierbar ist und die Wirkstofflösung durch die Mikrobohrungen applizierbar ist, wobei es sich bei den Mikrobohrungen um durchgehende Mikrobohrungen handelt und die Mikrobohrungen einen Durchmesser von 0,5 µm bis 10,0 µm, bevorzugt von 0,5 µm bis 5,0 µm und weiter bevorzugt von 1,0 µm bis 5,0 µm aufweisen und der Katheterballon pro mm² 1 bis 10 durchgehende Mikrobohrungen aufweist.

Der Begriff "durchgehend" wie hierin verwendet bedeutet, dass die Mikrobohrungen durch die Ballonhülle des Katheterballons hindurchgehen und somit die bei der Dilatation im Innern des Katheterballons befindliche Wirkstofflösung durch diese Mikrobohrungen von Innen nach außen austreten kann.

Dies ist anders bei Mikrosacklöchern. Dies sind Mikrobohrungen, welche nicht durchgehend sind, also die Ballonhülle des Katheterballons nicht vollständig durchdringen, sondern am Boden des Mikrosacklochs noch eine dünne Materialschicht oder eine dünne Membran verbleibt, so dass das Mikrosackloch eine nicht durchgehende Mikrobohrung ist und die bei der Dilatation im Innern des Katheterballons befindliche Wirkstofflösung erst dann austreten kann, wenn diese dünne Materialschicht oder diese dünne Membran aufgrund des aufgebauten Druckes bei der Dilatation platzt.

Erfindungsgemäß kann somit der Katheterballon anstelle der durchgehenden Mikrobohrungen auch mit **Mikrosacklöchern** versehen werden, wobei die Ballonhülle dann nicht vollständig durchbohrt ist (im Gegensatz zu den durchgehenden Mikrobohrungen). Als Mikrosackloch wird hier eine Bohrung bezeichnet, welche die Ballonhülle nicht vollständig durchdringt, also nur eine bestimmte Tiefe hat. Die Tiefe des Sackloches soll bevorzugt mindestens 70 % der Ballonwandstärke betragen, weiter bevorzugt mindestens 80 % und noch weiter bevorzugt mindestens 90% der Ballonwandstärke.

Die einzelnen Mikrosacklöcher haben vorzugsweise einen Innendurchmesser von 1,0 µm - 30,0 µm, bevorzugt von 1,5 µm - 25,0 µm, weiter bevorzugt von 2,0 µm - 20,0 µm, noch weiter bevorzugt von 2,3 µm - 15,0 µm, noch weiter bevorzugt von 2,5 µm - 10,0 µm, noch weiter bevorzugt von 2,7 µm - 8,0 µm, noch weiter bevorzugt von 2,9 µm - 6,0 µm und am meisten bevorzugt von 3,0 µm - 4,0 µm. Der Innendurchmesser bezieht sich auf eine idealisiert runde Form der Mikrosacklöcher. Sofern der Innendurchmesser über die Tiefe der Mikrobohrung nicht konstant sein sollte, wird als Innendurchmesser der kleinste Innendurchmesser bezeichnet.

Vorzugsweise werden die Mikrosacklöcher durch Abtragen mittels Laserstrahlung insbesondere mittels Ultra-Kurz-Pulslaser (UKP-Laser) und alternativ mittels Excimer-Laser hergestellt. Alternativ können die Sacklöcher auch durch Ionenstrahlbohren erzeugt werden.

Die verbleibende Ballonwandstärke am Boden der Mikrosacklöcher ist wie eine kleine Membran. Bei Beaufschlagung des Katheterballons mit Nominaldruck bzw. mit einer kurzen Druckspitze platzen die hauchdünnen verbliebenen Bohrungswandstärken (Membranen) auf, welche das Mikrosackloch vom inneren Volumen des Katheterballons trennen und öffnen so die Bohrungen und geben den Wirkstoff frei. D.h. durch das Platzen oder Bersten der verbliebenen Materialschicht am Boden eines Mikrosackloches wird aus dem Mikrosackloch eine durchgehende Mikrobohrung.

Die Mikrosacklöcher weisen gegenüber den durchgehenden Mikrobohrungen drei Vorteile auf:
a) Solange die verbliebene Ballonwandstärke in den Mikrosacklöchern nicht aufgeplatzt ist, wird verlässlich kein Wirkstoff freigesetzt.
b) Der Katheterballon kann mit einem höheren Druck beaufschlagt werden, bevor überhaupt Wirkstoff abgegeben wird.
c) Bei den Mikrosacklöchern können größere Bohrungsquerschnitte gebohrt werden. Trotz der großen Mikrosacklochquerschnitte kann ein hoher Ballondruck aufgebaut werden, d.h. der Ballon kann eine hohe Kraft zur Öffnung des stenotisierten Gefäßes aufbringen.

Die vorliegende Erfindung betrifft daher auch einen Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und/oder dilatierbar ist, wobei es sich bei den Mikrobohrungen um Mikrosacklöcher handelt, welche bis zu einer Tiefe von mindestens 70%, vorzugsweise mindestens 80%, weiter bevorzugt mindestens 90%, der Wandstärke der Ballonhülle des Katheterballons in die Ballonhülle hineinreichen.

Die vorliegende Erfindung betrifft ferner einen Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und/oder dilatierbar ist, wobei es sich bei den Mikrobohrungen um Mikrosacklöcher handelt, welche bis zu einer Tiefe von mindestens 70%, vorzugsweise mindestens 80%, weiter bevorzugt mindestens 90%, der Wandstärke der Ballonhülle des Katheterballons in die Ballonhülle hineinreichen und die zwischen Mikrosackloch und dem Inneren des Katheterballons befindliche Ballonhülle bei einem Druck von 8 MPa, bevorzugt von 6 MPa, weiter bevorzugt von 5 MPa platzt.

Die zwischen Mikrosackloch und dem Inneren des Katheterballons befindliche Ballonhülle wird hierin auch als dünne Materialschicht oder dünne Membran bezeichnet und ist das verbliebene Material der Ballonhülle am Boden eines jeden Mikrosacklochs. Dies ist das Material, welches bei einer durchgehenden Mikrobohrung im Vergleich zu einem Mikrosackloch entfernt worden ist. Diese am Boden eines Mikrosacklochs verbliebene Materialschicht wirkt eben wie eine Membran und stellt eine sogenannte Sollbruchstelle dar, d.h. diese Materialschicht platzt ab einem gewissen bei der Dilatation des Katheterballons aufgebauten Drucks.

Somit betrifft die vorliegende Erfindung auch einen Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und/oder dilatierbar ist, wobei es sich bei den Mikrobohrungen um Mikrosacklöcher handelt, welche bis zu einer Tiefe von mindestens 70%, vorzugsweise mindestens 80%, weiter bevorzugt mindestens 90%, der Wandstärke der Ballonhülle des Katheterballons in die Ballonhülle hineinreichen und berstbar sind. Zudem ist die Wirkstofflösung nach dem Bersten der berstbaren Mikrosacklöcher durch diese applizierbar.

Somit betrifft die vorliegende Erfindung auch einen Ballonkatheter mit einem Katheterballon, wobei der Katheterballon Mikrobohrungen aufweist und der Katheterballon mit einem Metallnetz überzogen ist und der Katheterballon mit einer Wirkstofflösung eines Antistenose-Mittels oder eines antiproliferativen, antiangiogenen oder antirestenotischen Wirkstoffs befüllbar und/oder dilatierbar ist, wobei es sich bei den Mikrobohrungen um Mikrosacklöcher handelt, welche bis zu einer Tiefe von mindestens 70%, vorzugsweise mindestens 80%, weiter bevorzugt mindestens 90%, der Wandstärke der Ballonhülle des Katheterballons in die Ballonhülle hineinreichen und einen Durchmesser von 2,0 µm bis 20,0 µm aufweisen und vorzugsweise der Katheterballon pro mm² 0,2 bis 6 Mikrosacklöcher aufweist.

Die Katheterballone mit Mikrosacklöchern sind vorzugsweise semi-compliant und besonders bevorzugt non-compliant Ballone. Das Material der non-compliant Ballone ist nicht dehnbar und kann durch hohe Ballondrücke besonders sicher zum Platzen gebracht werden.

Die Mikrosacklöcher können die gleichen Bohrungsdurchmesser wie die durchgehenden Mikrobohrungen haben, weisen bevorzugt aber etwas größere Bohrungsquerschnitte auf, da die verbleibende Bohrungswandstärke (Membran) in einem größeren Querschnitt einfacher, zum Bersten gebracht werden kann. Größere Bohrungsquerschnitte können auch deshalb realisiert werden, weil die Membran der Mikrosacklöcher selbst bei großen Bohrungsdurchmessern einen hohen Druckaufbau im Katheterballon ermöglicht bzw. eine unerwünschte Freigabe des Wirkstoffes bereits bei niedrigen Drücken verhindert.

Die einzelnen Mikrosacklöcher sind etwas größer als die durchgehenden Mikrobohrungen und haben erfindungsgemäß einen Innendurchmesser von 0,1 µm - 10,0 µm, bevorzugt von 1 µm - 6,0 µm, weiter bevorzugt von 2 µm - 5 µm. Der Innendurchmesser bezieht sich auf eine idealisiert runde Form der Mikrosacklöcher. Sofern der Innendurchmesser über die Tiefe des Sackloches nicht konstant sein sollte, wird als Innendurchmesser der kleinste Innendurchmesser bezeichnet.

Erfindungswesentlich sind daher folgende Merkmale für die Mikrosacklöcher:
a) Der Bohrungsdurchmesser bzw. Querschnitt der Mikrosacklöcher wird so groß gewählt, dass ein Platzen der verbleibenden Ballonwandstärke (Membran) bei einem erhöhten Druck (dem Nominaldruck) bzw. bei einer Druckspitze sicher erfolgt.
b) Eine Überdehnung des Gefäßes (durch einen zu stark druckgeweiteten Katheterballon) wird durch das Metallnetz, z.B. ein NiTi-Stützgerippe verhindert, welches nur eine maximale vorgegebene Größe, d.h. einen maximalen Durchmesser des dilatierten Katheterballons zulässt.

Die Anzahl an Mikrobohrungen pro mm² Ballonoberfläche beträgt vorzugsweise zwischen 0,2 und 10, weiter bevorzugt zwischen 0,5 und 9 oder zwischen 1 und 8. Beim Katheterballon beträgt die **Anzahl der durchgehenden Mikrobohrungen** pro mm² Oberfläche zwischen 0,5 und 10, vorzugsweise zwischen 1 und 10, weiter bevorzugt zwischen 1 und 8 Mikrobohrungen oder zwischen 4 und 5 Mikrobohrungen. Die **Anzahl der Mikrosacklöcher** pro mm² Oberfläche ist aufgrund der gegenüber den Mikrobohrungen typischerweise leicht größeren Bohrungsdurchmesser etwas geringer und beträgt zwischen 0,2 und 8, bevorzugt zwischen 0,2 und 6 oder zwischen 0,5 und 6 Sacklöcher, vorzugsweise zwischen 1 und 3 Sacklöcher. Am distalen und proximalen Ende des Katheterballons befinden sich weniger oder auch keine durchgehenden Mikrobohrungen bzw. Mikrosacklöcher mehr. Es ist bevorzugt, wenn sich die durchgehenden Mikrobohrungen bzw. Mikrosacklöcher möglichst gleichmäßig über das mittlere Segment des Katheterballons verteilen. Als mittleres Segment wird der Bereich des Katheterballons bezeichnet, der sich bei der Dilatation zu einer Zylinderform aufweitet. Die Mikrobohrungen können im Schachbrettmuster oder in Form einer dichtesten Kugelpackung in die Ballonhülle eingebracht werden.

Der Katheterballon wird vorzugsweise mittels einer **Kontrastmittellösung** expandiert, welche in den Balloninnenraum gepresst wird. Diese Lösung enthält auch einen oder mehrere Antirestenose-Wirkstoffe oder Antistenose-Mittel. Mittels dieser Lösung wird im Innern des Katheterballons ein Druck aufgebaut, der zuerst den Ballon entfaltet, ohne dabei die Ballonhülle zu dehnen und ab einem gewissen Druck, der vorzugsweise dem Nominaldruck entspricht, dehnt sich die Ballonhülle bei vollständig entfaltetem Ballon.

Die vorliegende Erfindung betrifft daher einen Ballonkatheter mit einem Katheterballon, wobei der Katheterballon Mikrobohrungen aufweist und mit einem Metallnetz überzogen ist und der Katheterballon im Innern mit einer Wirkstofflösung eines Antistenose-Mittels oder eines antiproliferativen, antiangiogenen oder antirestenotischen Wirkstoffs befüllbar ist, welche durch die Ballonhülle des Katheterballons applizierbar ist.

Die vorliegende Erfindung betrifft auch einen Ballonkatheter mit einem Katheterballon, wobei der Katheterballon Mikrobohrungen in Form von durchgehenden Mikrobohrungen oder Mikrosacklöchern aufweist und mit einem Metallnetz überzogen ist und der Katheterballon im Innern mit einer Wirkstofflösung eines Antistenose-Mittels oder eines antiproliferativen, antiangiogenen oder antirestenotischen Wirkstoffs befüllbar ist, welche durch die Ballonhülle des Katheterballons applizierbar ist.

Die Mikrobohrungen sind derart ausgestaltet, dass die Mikrobohrungen während der Entfaltung keine oder nur sehr geringe Mengen der Flüssigkeit, insbesondere der Kontrastmittellösung für die Ballondilatation passieren lassen. Beginnt jedoch nach vollständiger Entfaltung und bei weiterer Druckerhöhung sich die Ballonhülle eines "semi-compliant" oder "compliant"-Katherballons zu dehnen, dehnen sich damit auch die Mikrobohrungen, d.h. die Mikrobohrungen vergrößern ihren Innendurchmesser. Erst ab diesem Druck lassen die Mikrobohrungen eine nennenswerte Menge an Dilatationsflüssigkeit, d.h. insbesondere die Kontrastmittellösung oder die Wirkstofflösung mit oder ohne Kontrastmittel, aus dem Balloninneren nach außen treten.

Der erfindungsgemäße Ballonkatheter weist einen Katheterballon mit Mikrobohrungen auf, welche sich sozusagen erst ab einem bestimmten Druck innerhalb des Katheterballons öffnen und eine Lösung bevorzugt eine wässrige Lösung unter Druck freisetzen, welche vorzugsweise zeitgleich auch zur Dilatation des Katheterballons verwendet wird und zudem vorzugsweise ein Kontrastmittel und mindestens einen Antirestenose-Wirkstoff oder ein Antistenose-Mittel enthält. Ferner ist es möglich, eine bestimmtes Volumen dieser Lösung zu applizierten und damit aufgrund der bekannten Konzentration des Wirkstoffs in der Lösung eine genaue Menge an Wirkstoff in die Gefäßwand zu injizieren oder besser gesagt unter Druck zu spritzen. Dies wird zum ersten Mal auch eine Studie hinsichtlich der tatsächlich notwendigen geringsten Konzentration an Antirestenose-Wirkstoff ermöglichen, weil bisher noch keine Möglichkeit besteht, eine genau definierte Menge an Antirestenose-Wirkstoff zu applizieren und zudem auch noch direkt an die Wirkort zu bringen, d.h. in die Gefäßwand zu applizieren. Die wirkstoffenthaltenden Ballonbeschichtungen aus dem Stand der Technik verwenden, wenn auch nur lokal appliziert, wahrscheinlich immer noch eine unnötig hohe Wirkstoffdosis.

Die Möglichkeit, ein Antistenose-Mittel genau dosiert an der Wirkstelle gegebenenfalls in kurzen Zeitabständen mehrfach nacheinander zu applizieren, erlaubt erstmals eine Behandlung von kalzifizierten Stenosen und ermöglicht deren Auflösung.

Vorzugsweise öffnen sich die Mikrobohrungen bei einem "semi-compliant" oder "compliant"-Katheterballon ab einem **Balloninnendruck** von 2 MPa, weiter bevorzugt von 4 MPa, noch weiter bevorzugt von 5 MPa, noch weiter bevorzugt von 6 MPa, noch weiter bevorzugt von 7 MPa und am meisten bevorzugt von 8 MPa. Bei einem "non-compliant" Katheterballon geben die Bohrungen erst ab einem Balloninnendruck von 5 MPa, weiter bevorzugt von 8 MPa, noch weiter bevorzugt von 10 MPa, und am meisten bevorzugt von 12 MPa eine nennenswerte Medikamentendosis ab. Ab diesem Balloninnendruck sind die Ballone zur Applikation einer wässrigen Lösung und insbesondere einer wässrigen, mindestens einen Antirestenose-Wirkstoff oder ein Antistenose-Mittel enthaltenden Kontrastmittellösung geeignet. Allgemein lässt sich sagen, dass die erfindungsgemäßen Katheterballons bei einem Balloninnendruck von 2 bis 15 MPa zur Applikation einer Wirkstofflösung, bevorzugt einer wirkstoffenthaltenden Kontrastmittellösung oder einer Wirkstofflösung mit einem Kontrastmittel geeignet sind.

Setzt man der Dilatationsflüssigkeit und vorzugsweise der Kontrastmittellösung einen **Antirestenose-Wirkstoff** und/oder ein **Antistenose-Mittel** zu, so wird der Antirestenose-Wirkstoff und/oder das Antistenose-Mittel auf diese Weise direkt in die Gefäßwand und das umliegende Gewebe injiziert, weil die Abgabe der den Antirestenose-Wirkstoff und/oder das Antistenose-Mittel enthaltenden Lösung unter hohem Druck erfolgt. Damit wird der Antirestenose-Wirkstoff oder das Antistenose-Mittel direkt dorthin appliziert, wo er/es benötigt wird. Zudem ist ein großer Vorteil, dass sich der Wirkstoff nicht auf der Ballonoberfläche befindet, wo er leicht abgelöst werden kann, sondern als Lösung aus dem Innern des Ballons appliziert wird, wodurch es möglich ist, eine definierte Menge an Wirkstoff über ein definiertes Volumen an Wirkstofflösung zu applizieren. Bevorzugt ist daher die Applikation einer wirkstoffenthaltenden Kontrastmittellösung oder einer Wirkstofflösung, welche ein Kontrastmittel enthält, wobei diese Lösung auch zur Dilatation des Katheterballons verwendet wird.

Als Kontrastmittellösung für die Inflation des Katheterballons können die üblichen jodierten Kontrastmittel für die Angioplastie verwendet werden.

Als **Antirestenose-Wirkstoffe** werden antiinflammatorische, cytostatische, cytotoxische, antiproliferative, antimikrotubuli, antiangiogene, antineoplastische, antimigrative, athrombogene und antithrombogene Wirkstoffe eingesetzt.

Als antiinflammatorische, cytostatische, cytotoxische, antiproliferative, antimikrotubuli, antiangiogene, antineoplastische, antimigrative, athrombogene und antithrombogene Wirkstoffe können bevorzugt eingesetzt werden: Vasodilatatoren, Sirolimus (Rapamycin), Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, 8-□-Ergoline, Dimethylergoline, Agroclavin, 1-Allylisurid, 1-Allyltergurid, Bromergurid, Bromocriptin (Ergotaman-3',6',18-trione, 2-bromo-12'-hydroxy-2'-(1-methylethyl)-5'-(2-methylpropyl)-, (5'alpha)-), Elymoclavin, Ergocristin (Ergotaman-3',6',18-trione, 12'-hydroxy-2'-(1-methylethyl)-5'-(phenylmethyl)-, (5'-alpha)-), Ergocristinin, Ergocornin (Ergotaman-3',6',18-trione, 12'-hydroxy-2',5'-bis(1-methylethyl)-, (5'-alpha)-), Ergocorninin, Ergocryptin (Ergotaman-3',6', 18-triole, 12'-hydroxy-2'-(1-methylethyl)-5'-(2-methylpropyl)-, (5'alpha)- (9CI)), Ergocryptinin, Ergometrin, Ergonovin (Ergobasin, INN: Ergometrin, (8beta(S))-9,10-Didehydro-N-(2-hydroxy-1-methylethyl)-6-methyl-ergoline-8-carboxamid), Ergosin, Ergosinin, Ergotmetrinin, Ergotamin (Ergotaman-3',6',18-trione, 12'-hydroxy-2'-methyl-5'-(phenylmethyl)-, (5'-alpha)- (9CI)), Ergotaminin, Ergovalin (Ergotaman-3',6',18-trione, 12'-hydroxy-2'-methyl-5'-(1-methylethyl)-, (5'alpha)-), Lergotril, Lisurid (CAS-Nr.: 18016-80-3, 3-(9,10-Didehydro-6-methylergolin-8alpha-yl)-1,1-diethylharnstoff), Lysergol, Lysergsäure (D-Lysergsäure), Lysergsäureamid (LSA, D-Lysergsäureamid), Lysergsäurediethylamid (LSD, D-Lysergsäurediethylamid, INN: Lysergamid, (8ß)-9,10-Didehydro-N,N-diethyl-6-methyl-ergoline-8-carboxamid), Isolysergsäure (D-Isolysergsäure), Isolysergsäureamid (D-Isolysergsäureamid), Isolysergsäurediethylamid (D-Isolysergsäurediethylamid), Mesulergin, Metergolin, Methergin (INN: Methylergometrin, (8beta(S))-9,10-Didehydro-N-(1-(hydroxymethyl)propyl)-6-methyl-ergoline-8-carboxamid), Methylergometrin, Methysergid (INN: Methysergid, (8beta)-9,10-Didehydro-N-(1-(hydroxymethyl)propyl)-1,6-dimethyl-ergoline-8-carboxamid), Pergolid ((8β)-8-((Methylthio)methyl)-6-propyl-ergolin), Protergurid und Tergurid, Celecoxip, Thalidomid, Fasudil^{®,} Cyclosporine, SMC-Proliferation-lnhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C, IL1-ß-Inhibitor, Thymosin α-1, Fumarsäure und deren Ester, Calcipotriol, Tacalcitol, Lapachol, ß-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A und B, Paclitaxel und dessen Derivate wie 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, Gpllb/Illa-Plättchenmembranrezeptor, Faktor Xₐ-Inhibitor Antikörper, Interleukininhibitoren, Heparin, Hirudin, r-Hirudin, PPACK, Protamin, Natriumsalz der 2-Methylthiazolidin-2,4-dicarbonsäure Prourokinase, Streptokinase, Warfarin, Urokinase, Vasodilatatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vasiprost, Interferon α, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol, Vitamin B1, B2, B6 und B12, Folsäure, Tranilast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, D24851, SC-58125, Retinolsäure, Quinidin, Disopyrimid, Flecainid, Propafenon, Sotolol, Amidoron, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1 a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B und schwefelhaltige Aminosäuren wie Cystin sowie Salze, Hydrate, Solvate, Enantiomere, Racemate, Enantiomerengemische, Diastereomerengemische; Metaboliten, Prodrugs und Mischungen der vorgenannten Wirkstoffe.

Als Antirestenose-Wirkstoff sind insbesondere Paclitaxel und Paclitaxel-Derivate geeignet wie z.B. Taxotere, Baccatine, 7-xylosyl-10-Deacetyltaxol, Cephalomannin, 10-Deacetyl-7-epitaxol, 7-Epitaxol, 10-Deacetylcephalomannin, 7-Desoxy-docetaxol, 7,8-Zyklopropataxane, N-substituierte 2-Azetidone, 6,7-Epoxy-Paclitaxele, 6,7-modifizierte Paclitaxele, 10-Deacetoxytaxol, 10-Deacetyltaxol (aus 10-Deacetylbaccatin III), Phosphonooxy- und Karbonatderivate von Taxol, Taxol 2',7-Dinatrium-1,2-benzoldicarboxylat, 10-Deacetoxy-11,12-dihydrotaxol-10,12(18)-dien Derivate, 10-Deacetoxytaxol, Protaxol (2'-und/oder 7-O-Ester Derivate), 2'-und/oder 7-O-carbonatderivate von Paclitaxel, Fluortaxole, 9-Deoxotaxan, 13-Acetyl-9-deoxobaccatin III, 9-Deoxotaxol, 7-Deoxy-9-deoxotaxol, 10-Deacetoxy-7-deoxy-9-deoxotaxol, sulfoniertes 2'-Acryloyltaxol, sulfoniertes 2'-O-Acylsäuretaxolderivate, Succinyltaxol, 2'-gamma-Aminobutyryltaxol, 2'-Acetyltaxol, 7-Acetyltaxol, 7-Glycin-carbamat-taxol, 2'-OH-7-PEG(5000)-Carbamat-taxol, 2'-benzoyl Derivate von Paclitaxel, 2',7-Dibenzoyl-taxol, 2'-Acetyltaxol; 2',7-Diacetyltaxol; 2'-Succinyltaxol; 2'-(beta-alanyl)-Taxol, Ethylenglycol Derivate von 2'- Succinyltaxol, 2'-Glutaryltaxol, 2'-(N,N-dimethylglycyl)-Taxol, 2'-(2-(N,N-dimethylamino)propionyl)Taxol, 2'-Orthocarboxybenzoyl-taxol, 2'aliphatische Carboxylsäurederivate von Taxol, 2'-(N,N-Diethylaminopropionyl)taxol, 7-(N,N-Dimethylglycyl)taxol, 2',7-di-(N,N-Dimethylglycyl)taxol, 7-(N,N-Diethylaminopropionyl)taxol, 2',7-di(N,N-Diethylaminopropionyl)taxol, 2'-(L-Glycyl)taxol, 7-(L-Glycyl)taxol, 2',7-di(L-Glycyl)taxol, 2'-(L-Alanyl)taxol, 7-(L-Alanyl)taxol, 2',7-di(L-Alanyl)taxol, 2'-(L-Leucyl)taxol, 7-(L-Leucyl)taxol, 2',7-di(L-Leucyl)taxol, 2'-(L-Isoleucyl)taxol, 7-(L-Isoleucyl)taxol, 2',7-di(L-Isoleucyl)taxol, 2'-(L-Valyl)taxol, 7-(L-Valyl)taxol, 2'7-di(L-Valyl)taxol, 2'-(L-Phenylalanyl)taxol, 7-(L-Phenylalanyl)taxol, 2',7-di(L-Phenylalanyl)taxol, 2'-(L-Prolyl)taxol, 7-(L-Prolyl)taxol, 2',7-di(L-Prolyl)taxol, 2'-(L-Lysyl)taxol, 7-(L-Lysyl)taxol, 2',7-di(L-Lysyl)taxol, 2'-(L-Glutamyl)taxol, 7-(L-Glutamyl)taxol, 2',7-di(L-Glutamyl)taxol, 2'-(L-Arginyl)taxol, 7-(L-Arginyl)taxol, 2',7-di(L-Arginyl)taxol, (N-Debenzoyl-N-tert-(butoxycaronyl)-10-deacetyltaxol, Baccatin III, 10-Deacetylbaccatin III, Brevifoliol, Yunantaxusin, Taxusin, 14-beta-Hydroxy-10-deacetybaccatin III, Debenzoyl-2-acylpaclitaxelderivate, Benzoatpaclitaxelderivate, 18-Seitenketten-substituierte Paclitaxelderivate, chlorierte Paclitaxelanaloga, C4 Methoxyetherpaclitaxelderivate, Sulfonamidtaxanderivate, bromierte Paclitaxelanaloga, Girard-taxolderivate, Nitrophenylpaclitaxel, 10-Deacetylat substituierte Paclitaxelderivate, 14-beta-Hydroxy-10-deacetylbaccatin III Derivate, C7 Taxanderivate, 2-Debenzoyl-2-acyltaxanderivate, 2-Debenzoylpaclitaxelderivate, 2-Acylpaclitaxelderivate, 10-Deacetyltaxol A, 10-Deacetyltaxol B, 2-aroyl-4-acyl Paclitaxelanaloga, ortho-Ester Paclitaxelanaloga, Baccatin VII; Baccatin VI; Baccatin IV; 7-epi-Baccatin III; Baccatin V ; Baccatin I; Baccatin A; Epitaxol.

Des weiteren sind insbesondere Rapamycin (Sirolimus) und Rapamycin-Derivate bevorzugt wie z.B. Methylrapamycin, Biolimus A9, Deforolimus, Everolimus, Myolimus, Novolimus, Pimecrolimus, Ridaforolimus, Tacrolimus, Temsirolimus, Zotarolimus, 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamyc 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin, 40-O-(2-Hydroxy)ethoxycarbonylmethyl-rapamycin, 40-O-(3-Hydroxy)propyl-rapamycin, 40-O-(6-Hydroxy)hexyl-rapamycin, 40-O-[2-(2-Hydroxy)ethoxy]ethyl-rapamycin, 40-O-[(3S)-2,2-Dimethyldioxolan-3-yl]methyl-rapamycin, 40-O-[(2S)-2,3-Dihydroxyprop-1-yl]-rapamycin, 40-O-(2-Acetoxy)ethyl rapamycin, 40-O-(2-Nicotinoyloxy)ethyl-rapamycin, 40-O-[2-(N-Morpholino)acetoxy]ethyl-rapamycin, 40-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methylrapamycin, 40-O-(2-Aminoethyl)-rapamycin, 40-O-(2-Acetaminoethyl)-rapamycin, 40-O-(2-Nicotinamidoethyl)-rapamycin, 40-O-(2-(N- Methyl-imidazo-2'-ylcarbethoxamido)ethyl)-rapamycin, 40-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus)

Anstelle oder zusätzlich zu den Antirestenose-Wirkstoffen können auch Wirkstoffe zur Auflösung der Kalzifizierungen durch die Mikrobohrungen in der Ballonhülle des Katheterballons freigesetzt werden, die hier als Antistenose-Mittel bezeichnet werden. Beispiele für Antistenose-Mittel sind Entkalzifizierungsmittel, Dekalzifizierungsmittel sowie Stenoseabbaumittel.

Damit wird erstmals nicht die Folge einer Stenose durch Aufweiten des verengten Gefäßes sondern die Ursache, d.h. die kalzifizierte Stenose selbst behandelt.

Harte Kalzifizierungen bestehen aus Calciumcarbonat. Zur Auflösung von Calciumcarbonat können als Antistenose-Mittel, stets in niedriger körperverträglicher Konzentration, organische Säuren wie z.B. Milchsäure, Gluconsäure, Propionsäure, Essigsäure, Zitronensäure etc. zum Einsatz kommen, die mit Calciumcarbonat wasserlösliche Lactate, Gluconate, Propionate, Acetate oder Citrate bilden, oder auch anorganische Säuren. Mittels der durchgehend mikrogebohrten bzw. mit Mikrosacklöchern versehenen Katheterballone können die Antistenose-Mittel gezielt abgegeben werden. In einer einzigen angioplastischen Behandlung kann das Antistenose-Mittel genau dosiert in kurzen Zeitabständen mehrfach nacheinander immer wieder abgegeben werden, ohne dass der Ballonkatheter zwischendurch aus dem Gefäß entfernt werden muss. Die Abgabe in kurzen Zeitabstände ist erforderlich, um zwischendurch den Ballonkatheter zu deflatieren und den Blutfluss im Gefäß wieder herzustellen und somit eine Unterversorgung des Herzens zu vermeiden. Diese mehrfache Wirkstoffabgabe ist außerdem erforderlich, um bei den niedrigen Konzentrationen der Antistenose-Mittel bzw. aufgrund der Verdünnung durch das Blut eine ausreichende Wirkung zu erzielen.

Bei der häufig vorkommenden diffusen Koronarsklerosen, bei der mehrere über die Koronargefäße verteilte Stenosen vorliegen, ist es zudem möglich, nacheinander mit einem einzigen wirkstoffabgebenden Ballonkatheter alle kalzifizierten Stenosen zu behandeln und zumindest die leichten Kalzifizierungen weitgehend aufzulösen. Der mikrogebohrte Ballonkatheter wird hierzu an den kalzifizierten Gefäßstellen platziert und zur Wirkstoffabgabe mit Druck aufgeweitet wie hierin beschrieben.

Des Weiteren ist die Oberfläche des Katheterballons mit einem **Metallnetz** überzogen. Dieses Metallnetz sorgt für die gleichmäßige Dilatation des Katheterballons und damit für eine gleichmäßige Aufweitung des stenosierten Gefäßabschnitts und minimiert die Gefahr, das Gefäß zu überdehnen.

Im Stand der Technik wird fälschlich angenommen, dass bei der Setzung eines Stents in dem stenotisierten Gefäßabschnitt der Stent die Funktion hat, das Gefäß nach der Dilatation und Entfernung des Katheterballons mechanisch offen zu halten und dem Recoil, d.h. dem Gegendruck und der Rückstellkraft des Gefäßes entgegen zu wirken. Diese Ansicht wird hier als nicht zutreffend bezeichnet. Aufgrund der vorgenannten Ansicht werden Stents auch als Gefäßstützen bezeichnet, was ebenfalls unzutreffend ist.

Der Stent sorgt hauptsächlich für eine gleichmäßige Dilatation und nur zusätzlich für ein mechanisches Offenhalten des Gefäßabschnitts. Daher wurde mit der Einführung des Stents auch fälschlich angenommen, dass der Stent das Gefäß offen hält und damit einer Restenose vorbeugt. In Wirklichkeit verhindert der Stent aber die einseitige Überdehnung des Gefäßes bei der Ballondilatation wie im Folgenden dargelegt wird.

Beim ersten Inflatieren des Ballons legt sich der Stent sofort an die (verkalkte) Gefäßwand. Zu Beginn der Gefäßdilatation kann sich der Stent dann durchaus einseitig auf der nicht kalzifizierten Seite öffnen. Nach vollständiger Weitung der Stentstreben auf der einen Seite, müssen sich die Stege auf der gegenüberliegenden Seite dehnen. Somit wird das Gefäß gleichmäßig und schonend gedehnt. Der Ballon kann das Gefäß dadurch nicht einseitig überdehnen.

Die Festigkeit des Stents bzw. der Stentstreben ist damit nur wichtig, um Stegbrüche bei der Dilatation mit hohem Ballondruck zu vermeiden. Die hohe Festigkeit ist nicht erforderlich, um das geöffnete Gefäß zu stützen und ein Recoil, d.h. eine Wiederverengung des Gefäßes aufgrund von Rückstellkräften zu verhindern. Eine Stützfunktion und deren positive Wirkung kann aber nicht ganz ausgeschlossen werden. Hierzu reichen jedoch deutlich geringere Kräfte, zumal von außen keine Kräfte auf die Gefäße wirken, welche zu einer Wiederverengung führen würden und innerhalb der Gefäße der Blutdruck einer Gefäßverengung entgegenwirkt.

Da ein Stent zur Zeit noch aus nicht bioresorbierbaren Materialien und vor allem Metallen und Metalllegierungen besteht, stellt ein implantierter Stent einen dauerhaften Fremdkörper im Körper des Patienten dar, welcher auch noch nach längerer Zeit (einigen Jahren) Probleme bereiten kann, wie die Veröffentlichungen hinsichtlich der Spätthrombosen gezeigt haben.

Somit ist ein Vorteil der vorliegenden Erfindung, dass für die bestmögliche Verminderung oder Verhinderung einer Restenose die Implantation eines Stents nicht zwingend erforderlich ist.

Somit ist die gleichmäßige Dilatation des Katheterballons von besonderer Wichtigkeit. Dies kann aber gleichgut mit einem Metallnetz ähnlich einem stentähnlichen Geflecht erfolgen, welches fest mit dem Katheterballon verbunden ist und nicht wie ein Stent vom Katheterballon abgelöst werden kann.

Um daher eine gleichmäßige Dilatation und damit ein Aufbrechen von kalzifizierten Stenosen zu erreichen, ohne einen nicht kalzifizierten Gefäßabschnitt dabei zu überdehnen und auch gleichzeitig die Dehnung des "semi-compliant" bzw. "compliant" Katheterballons zu begrenzen, ist erfindungsgemäß der Katheterballon mit einem Metallnetz versehen, welches fest am Katheterballon montiert ist und nach erfolgter Dilatation mit dem Katheterballon auch wieder aus dem Patienten entfernt wird.

Erfindungsgemäß ist die **Ausgestaltung des Metallnetzes** so, dass es im deflatierten Zustand des Katheterballons möglichst eng an der Ballonoberfläche anliegt, damit der Durchmesser des Katheterballons im deflatierten Zustand (auch als komprimierter oder gefalteter Zustand bezeichnet) möglichst klein ist. Dennoch muss das Metallnetz derart ausgestaltet sein, dass es die Dilatation des Katheterballons zulässt, d.h. dass es sich wie ein Stent aufweiten lässt, ohne dass dabei Strebenbrüche auftreten. Die Aufweitung des Metallnetzes erfolgt dabei nicht durch Dehnung der Streben des Metallnetzes, sondern durch eine Aufweitung der Netzstruktur des Metallnetzes wie auch bei einem Stent.

Beim "semi-compliant" Katheterballon ist das Metallnetz beim Nominaldruck vorzugsweise noch nicht maximal aufgeweitet, sondern lässt bei diesem Druck noch eine geringe weitere Aufweitung zu, damit der vollständig entfaltete Katheterballon bei weiterer Druckerhöhung über den Nominaldruck hinaus sich in einem gewissen Rahmen noch dehnen kann.

Beim "non-compliant" PTCA-Katheterballon ist vorzugsweise beim Nominaldruck der Katheterballon als auch das Metallnetz maximal aufgeweitet.

Auch beim "compliant" PTCA-Katheterballon ist das Metallnetz beim Nominaldruck vorzugsweise maximal oder annähernd maximal gedehnt. Die Streben des Metallgitters müssen aber besonders engmaschig sein. Der Abstand zwischen zwei Metallstreben sollte im vollständig geweiteten Zustand kleiner 2,0 mm, vorzugsweise kleiner 1,5 mm und noch geeigneter kleiner 1,0 mm sein, damit der Ballon nicht zwischen den Streben hervortreten kann, wie es beispielsweise bei dem Produkt Chocolate^{®} der Firma TriReme Medical Inc. der Fall ist.

Dies bedeutet, dass sich bei der Inflation des Katheterballons das um den Katheterballon befindliche Metallnetz mit aufweitet. Bei dieser Aufweitung liegt das Metallnetz weiterhin möglichst eng an der Oberfläche des Katheterballons an. Dadurch wird eine gleichmäßige Aufweitung des Katheterballons erreicht und eine einseitige Überdehnung des Gefäßes, insbesondere eines Blutgefäßes unterbleibt.

Darüber hinaus führt diese gleichmäßige Dilatation des Katheterballons auch zu dem gewünschten Aufbrechen der Stenose und insbesondere einer kalzifizierten bis stark kalzifizierten Stenose.

Bei der Aufweitung des Metallnetzes während der Dilatation verlagern sich die Metallstreben des Metallnetzes relativ zu einander und erzeugen dadurch Scherkräfte wie sie auch bei der Aufweitung eines Stents entstehen. Diese Scherkräfte unterstützen auch das Aufbrechen von kalzifizierten Stenosen, verringern damit die Überdehnung des nicht kalzifizierten Gefäßabschnitts, vermeiden eine Dissektion und reduzieren dadurch die Restenoserate am besten.

Darüber hinaus kommt dem Metallnetz bei einem "semi-compliant" Katheterballon erfindungsgemäß noch eine weitere Funktion zu. Beim Nominaldruck ist das Metallnetz noch nicht maximal aufgeweitet und eine geringfügige weitere Aufweitung bis zu einem Maximaldurchmesser des Metallnetzes ist möglich.

Der Katheterballon kann daher vollständig entfaltet werden und hat danach noch die Möglichkeit sich bei weiterer Druckerhöhung zu dehnen, d.h. es findet eine weitere Vergrößerung des Durchmessers statt, welche durch die Dehnung der Hülle des Katheterballons entsteht und nicht mehr durch die Entfaltung des in Falten liegenden Katheterballons.

Bei dieser weiteren Aufweitung des "semi-compliant" Katheterballons, welche aufgrund der Dehnung des Materials des Katheterballons entsteht, dehnen sich auch die Mikrobohrungen, so dass diese ihren Innendurchmesser vergrößern und eine im Balloninneren enthaltene Lösung, welche vorzugsweise mindestens einen Antirestenose-Wirkstoff und/oder ein Antistenose-Mittel enthält, wird unter Druck in die Gefäßwand und das umliegende Gewebe injiziert. Auf gleiche Art und Weise kann auch die Öffnung der Mikrobohrungen in einem "compliant" PTCA-Katheterballon erfolgen.

Um diese weitere Vergrößerung des Katheterballons aufgrund Dehnung des "semi-compliant" und insbesondere des "compliant" Ballonmaterials zu begrenzen, kann dass Metallnetz nur bis zu einem definierten Durchmesser aufgeweitet werden, welches dann die weitere Vergrößerung des Katheterballons verhindert.

Das erfindungsgemäße Metallnetz beschränkt also die weitere Aufweitung des Katheterballons und legt damit einen Maximaldurchmesser für den maximal aufgeweiteten Katheterballon fest. Beim "semi-compliant" als auch beim "compliant" Katheterballon liegt dieser durch das Metallnetz vorgegebene maximale Durchmesser in dem Bereich, wo sich der "semi-compliant" Katheterballon nach Überschreiten des Nominaldruckes ausdehnen kann. Vorzugsweise liegt dieser maximale Durchmesser bei dem Durchmesser, den ein "semi-compliant" Katheterballon bei einem Druck hat, der über und vorzugsweise 0,5 MPa über dem Nominaldruck und unter 20 MPa vorzugsweise unter 18 MPa liegt. Bei einem solchen maximalen Durchmesser ist der Katheterballon vollständig entfaltet, so dass die Stenose und vor allem die kalzifizierte Stenose gleichmäßig aufgebrochen wurde und zudem ist die Ballonhülle leicht gedehnt, was zur Öffnung der Mikrobohrungen und zum Austritt der Lösung wie z.B. einer wirkstoffhaltigen physiologischen Lösung oder einer wirkstoffhaltigen Kontrastmittellösung führt.

Ferner ist das Metallnetz derart ausgestaltet, dass das Metallnetz von einem anfänglichen ersten Innendurchmesser mit der Dilatation des Katheterballons auf einem maximalen zweiten Innendurchmesser aufgeweitet und bei der Deflation des Katheterballons vorzugsweise wieder auf den ersten Innendurchmesser verkleinert werden kann oder auf einen Innendurchmesser verkleinert werden kann, der dem ersten Innendurchmesser möglichst nahe kommt.

Zudem ist bevorzugt, wenn das Metallnetz nicht derart großmaschig ist wie bei dem Produkt Chocolate^{®} der Firma TriReme Medical Inc., dass sich in den einzelnen Maschen des Metallnetzes Kissen ausbilden können, indem die Hülle des Katheterballons innerhalb der Maschen des Metallnetzes herausgedrückt wird und die Streben des Metallnetzes den Katheterballon zusammenschnüren, wobei sich Täler in der Ballonhülle entlang der Streben des Metallnetzes ausbilden. Bei dem vorgenannten Produkt aus dem Stand der Technik soll die Kontaktfläche von Ballonoberfläche mit der Gefäßwand maximiert werden, so dass die Ballonhülle aus den Maschen des Metallnetzes herausquellen soll, damit möglichst viel Wirkstoff von der Ballonoberfläche auf die Gefäßwand übertragen werden kann.

Da nicht vorgesehen aber auch nicht ausgeschlossen ist, dass der erfindungsgemäße Katheterballon mit einer wirkstoffenthaltenden Beschichtung versehen wird, ist eine möglichst große Kontaktfläche zwischen Ballonoberfläche und Gefäßwand für die vorliegende Erfindung unwichtig, weil der Wirkstoff aus dem Innern des Katheterballons durch die Mikrobohrungen (entweder durchgehende Mikrobohrungen oder durch Bersten der Membran geöffnete Mikrosacklöcher) hindurch appliziert wird.

Erfindungsgemäß ist das Metallnetz engmaschig und eher mit dem Metallnetz eines koronaren Stents zu vergleichen. Erfindungsgemäß ist bevorzugt, wenn sich die Ballonhülle nicht durch die Maschen des Metallnetzes herausdrücken kann.

Das Metallnetz wird aus einem elastischen Werkstoff, vorzugsweise aus dem superelastischen binärem Werkstoff Nickel-Titan (NiTi) hergestellt. Das Metallnetz kann aus dünnen NiTi Drähten geflochten oder gewebt werden. Vorzugsweise wird es aber aus einem NiTi-Rohr lasergeschnitten, da mit dem Laserschnitt das Gitterdesign bzw. die Designkonturen flexibel über die Länge des Katheterballons den Anforderungen angepasst werden können, wie fester Sitz auf dem Ballon am proximalen und distalen Ende, Begrenzung des maximalen aufgedehnten Durchmessers, etc. Zur Glättung der Oberfläche wird das Metallnetz elektropoliert aber nur so geringfügig, dass die Strebenkanten auf der Außenseite (abluminalen Gitterseite) scharfkantig bleiben und das Aufbrechen von kalzifizierten Stenosen erleichtern.

Da das Metallnetz am Katheterballon verbleibt und beim Entfernen des Katheterballons mit herausgezogen werden soll, muss es zum einen fest am Katheterballon verankert sein und zum anderen sich bei der Deflation des Katheterballons auch wieder möglichst weit zusammenziehen, damit es leicht zusammen mit dem Katheterballon wieder entfernt werden kann.

Bevorzugt ist daher, wenn das Metallnetz am distalen oder proximalen Ende und weiter bevorzugt am distalen und proximalen Ende fest mit dem Katheterballon verbunden ist beispielsweise indem dort das Netz die Ballonenden fest umschließt.

Ein Zusammenziehen des Metallnetzes bei der Deflation des Katheterballons kann erreicht werden, indem ein Metall oder eine Metalllegierung mit superelastischen Eigenschaften für das Metallnetz verwendet wird. Ein Metall oder eine Metalllegierung mit superelastischen Eigenschaften ist eine Legierung mit Formgedächtnis, wobei dieses Metall oder diese Metalllegierung bei einer bestimmten Temperatur oder einem bestimmten Temperaturbereich vorzugsweise eine bestimmte vorgegebene Form annimmt.

Eine geeignete Metalllegierung mit superelastischen Eigenschaften für das Metallnetz ist beispielsweise eine binäre Nickel-Titan-Legierung. Insbesondere bevorzugt ist eine Nickel-Titan-Legierung mit einem Gewichtsanteil von ca. 55% Nickel, die bei einer Transformationstemperatur von typischerweise 10°C bis 20°C unterhalb der Körpertemperatur (37°C) superelastische Eigenschaften aufweist. Entscheidend ist, dass Formgedächtnislegierungen wie z.B. NiTi superelastische Eigenschaften besitzen. Erfindungsgemäß können daher alle superelastischen Metalllegierungen und bevorzugt alle superelastischen Metalllegierungen mit Formgedächtnis eingesetzt werden.

Stellt man aus einem solchen Metall oder einer solchen Metalllegierung mit Formgedächtnis das Metallnetz für den Katheterballon so her, dass das Metallnetz beispielsweise bei 20°C die Form hat, dass es an dem komprimierten oder deflatierten Katheterballon möglichst eng anliegt, so wird dieses Netz bei mechanischer Verformung und bei einer Temperatur von oder im Bereich von 37°C versuchen, elastisch in diese Form zurückzukehren, welche ihm bei 20°C Temperatur vorgegeben worden ist.

Dies bedeutet, dass dem Metallnetz für eine Temperatur von 20°C eine Form vorgegeben wurde, welche möglichst eng am gefalteten oder komprimierten Katheterballon anliegt, damit sich dieser Katheterballon möglichst gut durch die Gefäße oder Blutbahnen führen oder schieben lässt. Wird dann der Katheterballon dilatiert, indem im Katheterballon ein entsprechender Druck aufgebaut wird, so gibt das Metallnetz diesem Druck nach und lässt sich unter diesem Zwang mechanisch elastisch verformen bis eine maximale Aufweitung des Metallnetzes erreicht ist, welche dann die weitere Aufdehnung des Katheterballons nach vollständiger Entfaltung bei weiterer Druckerhöhung verhindert.

Ist dann der Katheterballon vollständig entfaltet und hat sich unter dem weiter steigenden Druck aufgeweitet, um die durchgehenden Mikrobohrungen zu öffnen oder die Mikrosacklöcher durch Aufreißen der am Boden befindlichen Membran zu öffnen und eine Wirkstofflösung durch die geöffneten Mikrobohrungen freizusetzen, erfolgt die Deflation des Katheterballons, indem im Innern des Katheterballons ein Unterdruck erzeugt wird. Die Deflation des Katheterballons führt aber nicht zwangsläufig auch zu einem Zusammenziehen des Metallnetzes. Das Metallnetz zieht sich aufgrund der superelastischen Eigenschaften bei der Temperatur von 37°C wieder zusammen. Dies bedeutet, da im menschlichen Körper eine Temperatur von ca. 37°C herrscht, ist das Metallnetz bestrebt, die ursprünglich eng an den komprimierten Katheterballon anliegende Form wieder einzunehmen. Solange ein entsprechender Druck im Innern des Katheterballons eine mechanische Aufweitung des Metallnetzes erzwungen hat, konnten die Rückstellkräfte des Metallnetzes den Innendruck im Katheterballon nicht überwinden und das Metallnetz wurde und blieb aufgeweitet. Bei der Deflation des Katheterballons besteht dieser Überdruck im Innern des Katheterballons nicht mehr und die bei 37°C oder in der Nähe von 37°C wirkenden Rückstellkräfte des Metallnetzes kommen zum Tragen und bewirken ein Zusammenziehen des Metallnetzes weitgehend in die ursprüngliche Form. Damit verkleinert der Katheterballon mit dem Metallnetz seinen Durchmesser derart, dass er aus dem Gefäßsystem wieder herausgezogen werden kann. Als Durchmesser wird der maximale Durchmesser bezeichnet, der durch den Katheterballon und das Metallnetz erzeugt wird, was in der Regel dem Abstand zweier gegenüberliegender Streben des Metallnetzes mit maximalem Abstand zueinander entspricht.

Des Weiteren betrifft die vorliegende Erfindung Ballonkatheter mit einem Katheterballon, wobei der Katheterballon mit einem Metallnetz überzogen ist und Mikrobohrungen aufweist, welche erhältlich sind durch Behandlung eines Katheterballons ohne Mikrobohrungen mittels Laser zwecks Erzeugung der Mikrobohrungen und anschließendem Anbringen des Metallnetzes am Katheterballon.

Ein weiterer Aspekt der vorliegenden Erfindung ist auf die Herstellung von Ballonkatheter mit einem Katheterballon gerichtet, wobei der Katheterballon Mikrobohrungen aufweist und mit einem Metallnetz überzogen ist und der Katheterballon zur Aufnahme einer Wirkstofflösung eines Antistenose-Mittels oder eines antiproliferativen, antiangiogenen oder antirestenotischen Wirkstoffs ausgestaltet ist, welche durch die Mikrobohrungen applizierbar ist, umfassend die Schritte:
A) Bereitstellung eines Ballonkatheters mit einem Katheterballon ohne Kanäle, Bohrungen oder Öffnungen in der Ballonhülle des Katheterballons,
B) Einbringen von Mikrobohrungen mittels Laser in die Ballonhülle des Katheterballons und
C) Anbringung eines Metallnetzes über dem Katheterballon.

Zum Laserbohren der Katheterballone wird der Ballon mit einem inerten Gas aufgeblasen, d.h. entfaltet. Der entfaltete Ballon wird in einer Vorrichtung gehalten, um ihn präzise unter dem fokussierten Laserstrahl zu positionieren. Insbesondere die Fokuslage, d.h. die Lage des kleinsten Fokuspunktes ist für die Erzielung derart kleiner Mikrobohrungen entscheidend. Die hauchdünne Ballonhülle wird im Perkussionsverfahren, d.h. durch mehrere ultrakurze Laserpulse sukzessive durchbohrt. Die erforderlichen Pulsenergien sind je nach Ballonmaterial mit 0,05 bis 10 μJ sehr gering. Die Pulsfrequenzen liegen im kHz-Bereich, womit die Bohrungen in einem Bruchteil einer Sekunde eingebracht werden können. Die Laserstrahlwellenlänge wird je nach Ballonmaterial von 193 nm bis ca. 2000 nm gewählt.

Der erfindungsgemäße mikrogebohrte Ballonkatheter mit Metallnetz bietet entscheidende Vorteile gegenüber den Ballonkathetern des Standes der Technik:
1. Das Metallnetz mit superelastischen Eigenschaften dehnt sich mit dem Katheterballon und sichert eine gleichmäßige Aufweitung des Gefäßes und insbesondere kalzifizierter Gefäßabschnitte. Mit dem Ablassen des Druckes im Innern des Katheterballons zieht sich auch das Metallnetz mit Formgedächtnis wieder zusammen und wird mit dem Katheterballon aus dem Gefäß wieder entfernt.
2. Auch stark kalzifizierte Gefäßabschnitte werden gleichmäßig und homogen aufgedehnt, so dass die kalzifizierten Stenosen aufbrechen und eine Überdehnung eines nicht-kalzifizierten Gefäßbereichs vermieden wird.
3. Ist eine Stützung der Gefäßwandung z.B. wegen einer Dissektion erforderlich, kann anschließend ein ballonexpandierbarer Edelstahl- oder CoCr-Stent oder vorzugsweise bioresorbierbarer Polymer- bzw. Metallstent implantiert werden.
4. Grundsätzlich kann aber die Setzung eines Stents vermieden werden, sofern der Stent vorrangig die Funktion der gleichmäßigen Aufweitung der Stenose haben sollte und die dauerhafte Implantation eines Fremdkörpers in den Gefäßen des Patienten vermieden werden soll, wodurch auch die Gefahr von Spätthrombosen ausgeschlossen wird.
5. Erst ab einem gewissen Druck kann eine nennenswerte Menge der wirkstoffhaltigen Lösung aus dem Innern des Katheterballons durch die Mikrobohrungen hindurch direkt in die Gefäßwand gespritzt werden, so dass der Antirestenose-Wirkstoff nicht durch den Blutstrom weggespült wird, sondern direkt in den Wirkort injiziert wird.
6. Zudem ist es erstmals möglich, auf diese Weise eine genau definierte Menge eines Wirkstoffs zu applizieren.
7. Da sich der Wirkstoff nicht auf der Oberfläche des Katheterballons befindet, bestehen auch keine Probleme hinsichtlich der Haltbarkeit (shelf life), da der Wirkstoff erst im Moment der Dilatation in den Ballonkatheter eingebracht wird. Die erfindungsgemäßen mikrogebohrten Ballonkatheter mit Metallnetz sind daher grundsätzlich unbegrenzt haltbar.
8. Kurz vor der Dilatation kann ein geeigneter Wirkstoff ausgewählt werden und sogar vor Ort durch den behandelnden Kardiologen.
9. Da der erfindungsgemäße mikrogebohrte Ballonkatheter mit Metallnetz wirkstofffrei ist, bestehen auch nur geringere Zulassungshürden.
10. Es geht kein Wirkstoff beim Vorschieben des Ballonkatheters zur Stenose verloren.
11. Einmal eingeschoben kann der Ballonkatheter an mehreren stenosierten Gefäßabschnitten eingesetzt werden und immer wieder kann eine definierte Wirkstoffmenge abgegeben werden.
12. Der Wirkstoff wird förmlich in die Gefäßwand gespritzt und muss nicht hinein diffundieren, was im Blutgefäß immer zu Wirkstoffverlusten führt.

Ferner ergeben sich weitere klinische Anwendungsmöglichkeiten für den erfindungsgemäßen mikrogebohrten Ballonkatheter mit Metallnetz.

Insbesondere bevorzugt ist eine Kombination der Anwendung des erfindungsgemäßen Ballonkatheters mit einer nachfolgenden Implantation eines bioresorbierbaren Stents. Eine einmal mit dem erfindungsgemäßen Ballonkatheter geweitete und mit Wirkstoff behandelte Gefäßstenose kann anschließend mit einem vorzugsweise bioresorbierbaren Stent versorgt werden, wenn eine Gefäßstützung übergangsweise erforderlich ist.

Die bioresorbierbaren Stents aus Polymer oder Magnesium weisen generell eine geringe Materialfestigkeit auf und sind deshalb für die Ballondilatation weniger geeignet aus folgenden Gründen:

Die Stege legen sich zu Beginn der Dehnung an die Gefäßwand und folgen der ungleichmäßigen Gefäßdehnung, die sie aufgrund ihrer geringen Festigkeit nicht verhindern können. Wegen der geringen Materialfestigkeit können einige Stege sogar reißen. Nach Aufdehnung des bioresorbierbaren Stents beobachtet man typischerweise Stegüberlappungen, insbesondere eine ungleichmäßige Verteilung der Stege auf dem Umfang (Malapposition) und teilweise Stegbrüche. Eine gleichmäßige runde Gefäßdehnung kann nicht sichergestellt werden.

Bei "freier" Expansion der bioresorbierbaren Stents (an Luft) zeigen sie jedoch eine gleichmäßige bruchfreie Aufdehnung.

Genauso können sich die bioresorbierbaren Stents in einem vorgedehnten Gefäß (Predilatation) frei ohne große Kräfte und gleichmäßig entfalten und die erforderliche geringe Stützkraft gleichmäßig aufbringen.

Da das Medikament bereits über den mikrogebohrten Katheterballon appliziert wurde, ist eine Wirkstoffbeschichtung der bioresorbierbaren Stents nicht erforderlich, was die Produktzulassung sehr vereinfacht.

Die bioresorbierbaren Materialien haben bisher eine geringe Festigkeit und deshalb eine große Strebendicke mit der Folge, dass diese Stents im gecrimpten Zustand einen großen Durchmesser ("Profile") haben. Die bioresorbierbaren Stents können daher nur in relativ große Gefäßöffnungen bzw. Stenosen mit großem Lumen geschoben werden. Für mit dem erfindungsgemäßen Ballonkatheter vordilatierte Gefäße gibt es keine Beschränkungen.

Daher betrifft die vorliegende Erfindung auch ein Verfahren zur Applikation eines Antirestenose-Wirkstoffs oder Antistenose-Mittels durch Anwendung eines Katheterballons mit Mikrobohrungen und einem Metallnetz, wobei der Katheterballon mittels einer Kontrastmittellösung, die den Antirestenose-Wirkstoff oder das Anitstenose-Mittel enthält, inflatiert wird und sich bei steigendem Innendruck im Katheterballon zuerst der gefaltete Katheterballon entfaltet und nach vollständiger Entfaltung bei weiterer Druckerhöhung eine Dehnung der Ballonhülle erfolgt, wodurch sich die Mikrobohrungen öffnen und die Kontrastmittellösung, den Antirestenose-Wirkstoff oder das Antistenose-Mittel enthaltend, durch die Mikrobohrungen austritt, wobei die Dehnung der Ballonhülle durch das Metallnetz begrenzt wird. Dadurch kann eine definierte Menge eines Antirestenose-Wirkstoffs bzw. eines Antistenose-Mittels am Ort der Ballondilatation appliziert und vorzugsweise direkt in die Gefäßwand injiziert werden.

Die vorliegende Erfindung betrifft auch ein Verfahren zur Applikation einer Wirkstofflösung eines Antistenose-Mittels oder eines antiproliferativen, antiangiogenen oder antirestenotischen Wirkstoffs vorzugsweise einer Wirkstofflösung enthaltend ein Kontrastmittel oder eine wirkstoffenthaltende Kontrastmittellösung umfassend die Schritte:
A) Bereitstellung eines Ballonkatheters mit einem Katheterballon, wobei der Katheterballon Mikrobohrungen aufweist und mit einem Metallnetz überzogen ist und der Katheterballon zur Aufnahme einer Wirkstofflösung ausgestaltet ist, welche durch die Mikrobohrungen applizierbar ist,
B) Dilatation des Katheterballons mittels der Wirkstofflösung bis zu einem Druck, der die Mikrobohrungen öffnet, um ein vorbestimmtes Volumen an Wirkstofflösung zu applizieren und
C) Aufrechterhaltung des Drucks bis das vorbestimmte Volumen an Wirkstofflösung appliziert ist.

### Figurenbeschreibung

- Figur 1:: zeigt einen Katheterballon im komprimierten oder inflatierten Zustand, der in Falten gelegt ist. Der Kreis um den komprimierten oder deflatierten Katheterballon bezeichnet die idealisierte runde Form des Katheterballons zwecks Bestimmung des Durchmessers.
- Figur 2:: Flüssigkeitsabgabe von non-compliant Ballonen Figur 2 zeigt für einen non-complaint Ballon die Durchflussmenge von Wasser innerhalb von 30 Sekunden für 200, 400 und 600 Bohrungen mit einem Durchmesser von 2,0 µm ± 0,5 µm.
- Figur 3:: Flüssigkeitsabgabe von semi-compliant Ballonen Figur 3 zeigt für einen semi-complaint Ballon die Durchflussmenge von Wasser innerhalb von 30 Sekunden für 200 und 600 Bohrungen mit einem Durchmesser von 2,0 µm ± 0,5 µm.

### Beispiele

### Beispiel 1: Herstellung von Katheterballonen mit durchgehenden Mikrobohrungen und Flüssigkeitsabgabe

Der Katheterballone werden z.B. mittels Stickstoff inflatiert und mit einem Ultra-Kurzpuls-Laser (UKP-Laser) werden durchgehende Mikrobohrungen in der Ballonhülle erzeugt. Die Mikrobohrungen verlaufen durch die Ballonhülle senkrecht in Richtung Längsachse des Katheterballons. Die Mikrobohrungen besitzen einen Durchmesser von 2,0 µm ± 0,5 µm.

Die Flüssigkeitsabgabe von non-compliant und semi-compliant Ballonen wurde in Abhängigkeit der Anzahl der Bohrungen und des Flüssigkeitsdruckes gemessen.

### Beispiel 2: Herstellung von Katheterballonen mit Mikrosacklöchern und Flüssigkeitsabgabe

Der Katheterballone werden z.B. mittels Stickstoff inflatiert und zunächst einmal die Ballonwandstärke mit einer Messgenauigkeit kleiner 1 µm vermessen. Die Ballonwandstärke ist entscheidend für die Einstellung des Laserabtragsprozesses bzw. die verbleibende Membranwandstärke auf der Balloninnenseite.

Die Bearbeitung erfolgt mittels eines Ultra-Kurzpuls-Lasers. Der Laserstrahldurchmesser im Laserstrahlfokus wird durch optische Elemente auf einen Durchmesser von 10 µm eingestellt. Mit jedem Laserstrahlpuls wird sukzessive eine kleine definierte Schicht der Ballonwandstärke abgetragen, so dass eine zylindrische Bohrung mit einem Durchmesser von 10 µm entsteht. Die Anzahl der Laserpulse bestimmt die Tiefe des Mikrosackloches bzw. die verbleibende Wandstärke (Membran) am Bohrungsgrund. Je nach zuvor gemessener Ballonwandstärke wird die Anzahl der Laserpulse so gewählt, dass eine Membranwandstärke von 3-4 µm auf der Balloninnenseite verbleibt.

Ab einem Flüssigkeitsinnendruck von 4-5 bar (4-5 MPa) platzt die Membran, so dass eine Flüssigkeitsabgabe aus dem Katheterballon erfolgt.

### Beispiel 3: Bestimmung des applizierten Volumens an Dilatationslösung

Zur Bestimmung des applizierten Volumens einer Dilatationslösung wurden ein non-complaint Ballon mit jeweils 200, 400 und 600 durchgehende Mikrobohrungen sowie ein semi-complaint Ballon mit jeweils 200 und 600 durchgehenden Mikrobohrungen untersucht. Die Mikrobohrungen hatten jeweils einen Durchmesser von 2,0 µm ± 0,5 µm. Als Dilatationslösung dient in der Praxis die hierin beschriebene Wirkstofflösung oder vorzugsweise die wirkstoffenthaltende Kontrastmittellösung. In Beispiel 3 wurde aus Praktikabilitätsgründen gereinigtes Wasser (destilliertes Wasser) verwendet.

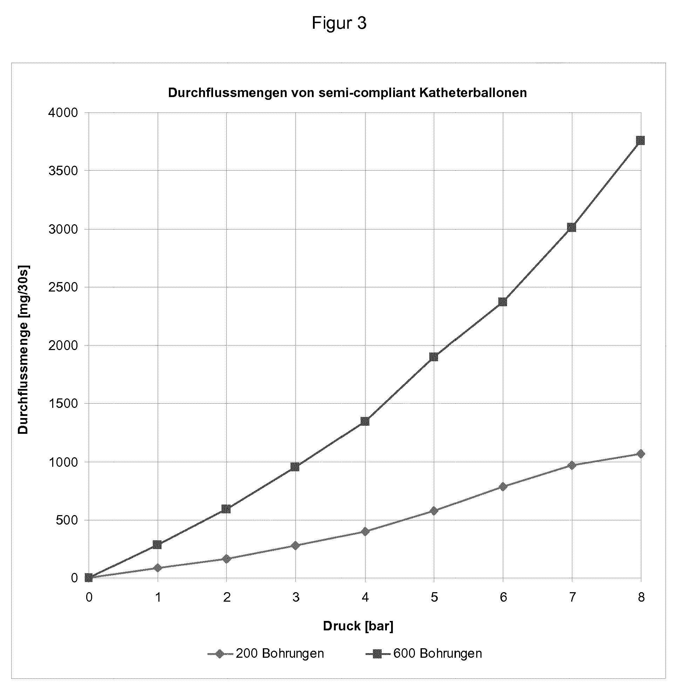

Die verwendeten erfindungsgemäßen Katheterballone wurden dazu schrittweise bis 8 bar (8 MPa) mit Wasser als Dilatationslösung dilatiert. Jeweils bei 1 bar (1 MPa), 2 bar (2 MPa), 3 bar (3 MPa), 4 bar (4 MPa), 5 bar (5 MPa), 6 bar (6 MPa), 7 bar (7 MPa) und 8 bar (8 MPa) wurde über einen Zeitraum von 30 Sekunden das Volumen an applizierter Dilatationslösung bestimmt. Dieser Versuch wurde dreimal wiederholt. Die Ergebnisse sind in den Figuren 2 und 3 dargestellt.

Aus Figur 2 ist für einen non-complaint Ballon zu entnehmen, dass bei 8 bar (8 MPa) innerhalb einer Dilatationszeit von 30 Sekunden bei einer Gesamtzahl von 200 Mikrobohrungen pro Ballon 78 mg Dilatationslösung appliziert werden. Die durchgehenden Mikrobohrungen weisen einen Durchmesser im Bereich von 1,5 µm bis 2,5 µm auf. Bei 400 Mikrobohrungen pro Ballon sind es bereits 283 mg und bei 600 Mikrobohrungen pro Ballon 656 mg (s. Figur 2). Die entsprechenden Werte für einen semi-complaint Ballon sind in Figur 3 dargestellt.

Im Vergleich der Figuren 2 und 3 ist die unterschiedliche Skalierung der Y-Achse (Durchflussmenge) zu beachten. Die semi-complaint Ballone geben deutlich mehr Flüssigkeit als die non-complaint Ballone ab, da sich die Bohrungen unter Druck mehr weiten. Die semi-compliant Ballone müssen daher mit entsprechend weniger durchgehenden Mikrobohrungen versehen werden oder sind für größere Flüssigkeits- d.h. Wirkstoffabgaben geeignet z.B. zur Abgabe der Antistenose-Mittel, die zur Wirksamkeit in größeren Mengen abgegeben werden müssen.

### Beispiel 4: Verwendung eines erfindungsgemäßen Ballonkatheters

Eingesetzt wird ein kommerziell erhältlicher üblicher PTCA-Ballonkatheter. Der Katheterballon ist unbeschichtet und besteht aus Polyamid (Handelsname Nylon) oder PET. Es handelt sich um einen "non-compliant"-Katheterballon, welcher praktisch unelastisch ist. Die Vergrößerung des Durchmessers von diesem "non-compliant"-Katheterballon beruht auf der Entfaltung des Katheterballons, ohne dass dabei die Materialdehnung der Hülle des Katheterballons eine Rolle spielt. Nach vollständiger Entfaltung kann durch weitere Erhöhung des Innendrucks im Katheterballon eine minimale Vergrößerung des Durchmessers des Katheterballons erreicht werden.

Der Katheterballon (ø 3.0 x 20 mm) wird z.B. mittels Stickstoff inflatiert und mit einem UKP-Laser werden durchgehende Mikrobohrungen in der Ballonhülle erzeugt. Die Mikrobohrungen verlaufen durch die Ballonhülle senkrecht in Richtung Längsachse des Katheterballons. Die durchgehenden Mikrobohrungen besitzen einen Durchmesser von 1,7 µm ± 0,3 µm. Pro mm² Oberfläche des vollständig expandierten Katheterballons werden 1,5 Mikrobohrungen erzeugt.

Der so bearbeitete Katheterballon wird danach im vollständig deflatierten Zustand mit einem eng anliegenden Metallnetz aus einer Nickel-Titan-Legierung (wie z.B. Nitinol^{®}) überzogen, welches am distalen und am proximalen Ende des Katheterballons fest mit dem Katheterballon verbunden wird, indem eine Schlaufe des Metallnetzes fest um das distale Ballonende und eine weitere Schlaufe fest um das proximale Ballonende gewickelt wird. Im Bereich zwischen dem distalen und dem proximalen Ende des Katheterballons ist das Metallnetz wie ein Stent mit netzartigen Streben ausgebildet, so dass sich dieser Bereich bei der Dilatation des Katheterballons aufweiten kann.

Das Metallnetz aus z.B. Nitinol^{®} vergrößert den Durchmesser des vollständig deflatierten und gefalteten Katheterballons nur unwesentlich. Der vollständig deflatierte und gefaltete Katheterballon ohne Metallnetz weist einen Durchmesser von 0,8 mm auf, wobei der vollständig deflatierte und gefaltete Katheterballon mit Metallnetz einen Durchmesser von 1,0 mm aufweist.

Der Katheterballon wird wie üblich beim Patienten über die Leiste bis zur Herzregion über einen Führungsdraht vorgeschoben und im stenosierten und kalzifizierten Gefäßabschnitt platziert. Nun erfolgt die Dilatation des Katheterballons indem eine wässrige Kontrastmittellösung des Kontrastmittels lopromid in das Innere des Katheterballons gepumpt wird. Die Kontrastmittellösung ist kommerziell erhältlich unter der Bezeichnung Ultravist^{®}. Der Kontrastmittellösung wurde der Wirkstoff Paclitaxel in einer Konzentration von 0,5 - 2,0 µg pro ml zugesetzt.

Mit steigendem Druck im Innenraum des Katheterballons beginnt sich der Katheterballon zu entfalten, wobei die Mikrobohrungen noch derart verschlossen sind, dass kein nennenswerter Austritt von paclitaxelhaltiger Kontrastmittellösung zu verzeichnen ist. Bei weiterer Druckerhöhung vergrößert der Katheterballon seinen Durchmesser weiterhin gleichmäßig durch das ihn umgebende Nitinolnetz, welches sich in gleichem Maße aufweitet wie der Katheterballon. Dadurch wird die kalzifizierte Stenose wirksam aufgebrochen und eine Überdehnung des nicht kalzifizierten Gefäßbereiches wirksam vermieden. Bei einem Druck von 6,0 MPa ist der Katheterballon vollständig entfaltet (Nominaldruck). Nun wird der Druck im Innern des Katheterballons auf 8 MPa erhöht. Dies führt dazu, dass die paclitaxelhaltige Kontrastmittellösung unter Druck aus den durchgehenden Mikrobohrungen strömt und regelrecht in die Gefäßwand injiziert wird. Zudem ist bei diesem Druck das Metallnetz maximal aufgeweitet und begrenzt mechanisch eine weitere Dehnung des Materials der Ballonhülle, falls der Druck im Katheterballon noch weiter erhöht werden sollte, so dass keine weitere Vergrößerung des Durchmessers des Katheterballons mehr stattfinden kann. Über einen Zeitraum von 20 Sekunden wird eine Medikamentendosis von ca. 2 bis 10 µg Paclitaxel pro Millimeter Gefäßwandlänge (entsprechend 0,20 bis 1 µg pro mm² Gefäßwandfläche) in die Gefäßwand gespritzt.

Danach wird der Druck im Ballon auf 0,5 MPa gesenkt, der Katheterballon leicht bewegt und erneut dilatiert bis ein Druck von 8 MPa erreicht ist. Über einen Zeitraum von weiteren 20 Sekunden wird erneut eine Medikamentendosis von ca. 2 bis 10 µg Paclitaxel pro Millimeter Gefäßwandlänge appliziert.

Nach der zweiten Dilatation wird der Druck im Katheterballon wieder abgelassen. Mit abnehmendem Druck beginnt auch der Katheterballon sich wieder zu verkleinern und in Falten zu legen. Das ihn umgebende Metallnetz zieht sich aufgrund der Rückstellkräfte ebenfalls wieder zusammen und liegt weiterhin an der Oberfläche des Katheterballons an. Eine weitgehende Deflation des Katheterballons wird abschließend durch Erzeugung eines Unterdrucks im Innern des Katheterballons erreicht. Da dem Metallnetz durch Wärmebehandlungsschritte die ursprüngliche eng am vollständig gefalteten und komprimierten Katheterballon anliegende Form gegeben wurde, strebt das Metallnetz bei Wegfall des Drucks im Innern des Katheterballons in die ursprüngliche Form und legt sich wieder weitestgehend eng an den deflatierten Katheterballon an und kann so zusammen mit dem Katheterballon leicht aus dem Patienten wieder entfernt werden.

Sollten zudem mehrere stenosierte Gefäßabschnitte desselben Patienten behandelt werden, so kann dies sogar unter Verwendung desselben Katheterballons erfolgen und unter gezielter Medikamentenabgabe durch die Mikrobohrungen hindurch an verschiedenen Stellen im Gefäßsystem.

## Patentansprüche

1. Ballonkatheter mit einem Katheterballon, wobei der Katheterballon Mikrobohrungen aufweist und mit einem Metallnetz überzogen ist und der Katheterballon mit einer Wirkstofflösung befüllbar und dilatierbar ist und ein vorbestimmtes Volumen der Wirkstofflösung durch die Mikrobohrungen während der Dilatation freigesetzt wird, wobei es sich bei der Wirkstofflösung um eine Lösung eines Antistenose-Mittels oder eines antiproliferativen, antiangiogenen oder antirestenotischen Wirkstoffs handelt.

2. Ballonkatheter gemäß Anspruch 1, wobei es sich bei dem Katheterballon um einen non-compliant oder semi-compliant oder compliant Katheterballon handelt.

3. Ballonkatheter gemäß Anspruch 1 oder 2, wobei das Metallnetz aus einem superelastischen Metall oder einer superelastischen Metalllegierung besteht.

4. Ballonkatheter gemäß einem der Ansprüche 1 - 3, wobei das Metallnetz aus einer Nickel-Titan-Legierung besteht.

5. Ballonkatheter gemäß einem der Ansprüche 1 - 4, wobei das Metallnetz von einem anfänglichen ersten Innendurchmesser mit der Dilatation des Katheterballons auf einen maximalen zweiten Innendurchmesser aufweitbar und bei der Deflation des Katheterballons wieder auf den ersten Innendurchmesser verkleinerbar ist.

6. Ballonkatheter gemäß einem der Ansprüche 1 - 5, wobei das Metallnetz nach maximaler Aufweitung die weitere Inflation des Katheterballons bei weiter steigendem Innendruck verhindert.

7. Ballonkatheter gemäß einem der Ansprüche 1 - 6, wobei der atheterballon pro mm² Oberfläche zwischen 0,2 und 10 Mikrobohrungen aufweist.

8. Ballonkatheter gemäß einem der Ansprüche 1 - 7, wobei es sich bei den Mikrobohrungen um durchgehende Mikrobohrungen oder um Mikrosacklöcher handelt.

9. Ballonkatheter gemäß Anspruch 8, wobei die durchgehenden Mikrobohrungen einen Durchmesser von 0,5 µm - 5,0 µm und die Mikrosacklöcher einen Durchmesser von 2,0 µm bis 20,0 µm haben.

10. Ballonkatheter gemäß Anspruch 8 oder 9, wobei der Katheterballon pro mm² Oberfläche zwischen 0,2 und 6 Mikrosacklöcher oder zwischen 1 und 10 durchgehende Mikrobohrungen aufweist.

11. Ballonkatheter gemäß einem der Ansprüche 1 - 10, wobei die Mikrobohrungen im Katheterballon mittels Laserbehandlung hergestellt wurden.

12. Ballonkatheter gemäß einem der Ansprüche 1 - 11, wobei die Mikrobohrungen in dem Katheterballon bei einem Balloninnendruck von 2 bis 15 MPa zur Applikation der Wirkstofflösung geeignet sind.

13. Ballonkatheter gemäß Anspruch 1, wobei als Antirestenose-Wirkstoff ein antiinflammatorischer, cytostatischer, cytotoxischer, antiproliferativer, antimikrotubuli, antiangiogener, antineoplastischer, antimigrativer, athrombogener oder antithrombogener Wirkstoff eingesetzt wird.

## Claims

1. Balloon catheter with a catheter balloon, wherein the catheter balloon has micro drillings and is covered with a metal mesh, and the catheter balloon is fillable with an active agent solution and is dilatable and a predefined volume of the active agent solution is released through the micro drillings during the dilatation, wherein the active agent solution is a solution of an anti-stenotic agent or an anti-proliferative, anti-angiogenic or anti-restenotic agent.

2. Balloon catheter according to claim 1, wherein the catheter balloon is a non-compliant or a semi-compliant or a compliant catheter balloon.

3. Balloon catheter according to claim 1 or 2, wherein the metal mesh is made of a superelastic metal or a superelastic metal alloy.

4. Balloon catheter according to any one of claims 1 - 3, wherein the metal mesh is made of a nickel-titanium-alloy.

5. Balloon catheter according to any one of claims 1 - 4, wherein the metal mesh is dilatable from an initial first inner diameter to a maximum second inner diameter with the dilatation of the catheter balloon and with deflation of the catheter balloon the size of the metal mesh is reduced to the first inner diameter again.

6. Balloon catheter according to any one of claims 1 - 5, wherein after maximal dilatation the metal mesh prevents further dilatation of the catheter balloon with increasing internal pressure.

7. Balloon catheter according to any one of claims 1 - 6, wherein the catheter balloon has between 0.2 and 10 micro drillings per mm² of its surface.

8. Balloon catheter according to any one of claims 1 - 7, wherein the micro drillings are continuous drillings or micro blind holes.

9. Balloon catheter according to claim 8, wherein the continuous drillings have a diameter of 0.5 µm - 5.0 µm and the micro blind holes have a diameter of 2.0 µm to 20.0 µm.

10. Balloon catheter according to claim 8 or 9, wherein the catheter balloon has between 0.2 and 6 micro blind holes or between 1 and 10 continuous drillings per mm² of its surface.

11. Balloon catheter according to any one of claims 1 - 10, wherein the micro drillings in the catheter balloon are made using laser treatment.

12. Balloon catheter according to any one of claims 1 - 11, wherein the micro drillings in the catheter balloon are suitable for administration of the active agent solution at a balloon internal pressure of 2 to 15 MPa.

13. Balloon catheter according to claim 1, wherein as anti-stenotic agent an antiinflammatory, cytostatic, cytotoxic, anti-proliferative, anti-microtubule, anti-angiogenic, anti-neoplastic, anti-migrative, athrombogenic or anti-thrombogenic agent is used.

## Revendications

1. Un cathéter à ballonnet avec un ballonnet à cathéter, dans lequel le ballonnet à cathéter a des micro-trous et est revêtu d'un treillis métallique, et le ballonnet à cathéter peut être rempli avec une solution d'agent actif et peut être dilaté, et un volume prédéfini de la solution d'agent actif peut être relâchée à travers les micro-trous pendant la dilatation, où la solution d'agent actif est une solution d'agent anti-sténotique ou d'agent antiprolifératif, anti-angiogénique ou anti-resténose.

2. Le cathéter à ballonnet selon la revendication 1, dans lequel le ballonnet à cathéter est un ballonnet à cathéter non-conforme, ou semi-conforme ou conforme.

3. Le cathéter à ballonnet selon la revendication 1 ou 2, dans lequel le treillis métallique est composé d'un métal super-élastique ou un alliage métallique super-élastique.

4. Le cathéter à ballonnet selon une quelconque des revendications 1 - 3, dans lequel le treillis métallique est composé d'un alliage nickel - titane.

5. Le cathéter à ballonnet selon une quelconque des revendications 1 - 4, dans lequel le treillis métallique est gonflable d'un premier diamètre intérieur initial à un deuxième diamètre intérieur maximum avec la dilatation du ballonnet à cathéter et réductible avec la déflation du ballonnet à cathéter au premier diamètre intérieur.

6. Le cathéter à ballonnet selon une quelconque des revendications 1 - 5, dans lequel le treillis métallique empêche après dilatation maximale la dilatation ultérieure du ballonnet à cathéter suite à l'augmentation de la pression interne.

7. Le cathéter à ballonnet selon une quelconque des revendications 1 - 6, dans lequel le ballonnet à cathéter a de 0,2 à 10 micro-trous per mm² de sa surface.

8. Le cathéter à ballonnet selon une quelconque des revendications 1 - 7, dans lequel les micro-trous sont des micro-trous continus ou des micro-trous borgnes.

9. Le cathéter à ballonnet selon la revendication 8, dans lequel les micro-trous continus ont un diamètre de 0,5 µm - 5,0 µm et les micro-trous borgnes ont un diamètre de 2,0 µm à 20,0 µm.

10. Le cathéter à ballonnet selon la revendication 8 ou 9, dans lequel le ballonnet à cathéter a entre 0,2 à 6 micro-trous borgnes ou entre 1 et 10 micro-trous continus par mm² de sa surface.

11. Le cathéter à ballonnet selon une quelconque des revendications 1 - 10, dans lequel les micro-trous dans le ballonnet à cathéter ont été réalisés par traitement au laser.

12. Le cathéter à ballonnet selon une quelconque des revendications 1 - 11, dans lequel les micro-trous dans le ballonnet à cathéter sont appropriés pour l'administration de la solution d'agent actif à une pression interne du ballonnet de 2 à 15 MPa.

13. Le cathéter à ballonnet selon la revendication 1, où un agent anti-inflammatoire, cytostatique, cytotoxique, anti - prolifératif, anti - microtubules, anti - angiogènique, anti - néoplasique, anti - migratoire, athrombogénique ou anti - thrombogénique est utilisé en tant qu'agent anti - resténose.
